# EUROPÄISCHE PATENTSCHRIFT

(11) **EP 1 191 041 B1**
(45) Veröffentlichungstag und Bekanntmachung des Hinweises auf die Patenterteilung: **15.12.2004**
(21) Anmeldenummer: 01122452.4
(22) Anmeldetag: 20.09.2001
(51) Int. Cl.: C08F 2/44, A61K 7/00

(54) **Farbmittelhaltige wässrige Polymerdispersion**
Aqueous polymer dispersions containing colorants
Dispersions aqueuses de polymères contenant des colorants

(30) Priorität: 21.09.2000 DE 10046927
(43) Veröffentlichungstag der Anmeldung: 27.03.2002
(73) Patentinhaber: BASF AKTIENGESELLSCHAFT, 67056 Ludwigshafen (DE)
(72) Erfinder: Habeck, Thorsten, 67149 Meckenheim (DE); Mathauer, Klemens, 67063 Ludwigshafen (DE); Wünsch, Thomas, 67346 Speyer (DE); Westenfelder, Horst, 67435 Neustadt (DE); Ichihara, Hideyuki, Odawara, Kanagawa 250-0874 (JP); Ikeda, Takahiro, Nishinomiya Hyogo 663-8141 (JP); Dausch, Wilma M., 67117 Limburgerhof (DE)
(74) Vertreter: Kinzebach, Werner, Dr.

(56) Entgegenhaltungen:
- WO-A-83/00151
- DE-A- 19 811 314

## Beschreibung

Die vorliegende Erfindung betrifft farbmittelhaltige wässrige Polymerdispersionen feinteiliger, farbmittelhaltiger Polymerisatteilchen und daraus hergestellte farbmittelhaltige Polymerpulver sowie deren Verwendung in kosmetischen Mitteln.

Farbmittelhaltige Polymerdispersionen und daraus hergestellte Polymerpulver sind aus der WO 99/01967 bekannt. Sie enthalten wenigstens ein Farbmittel, das in den Polymerisatteilchen der farbmittelhaltigen Polymerdispersion in weitgehend homogener Verteilung vorliegt.

In den farbmittelhaltigen Polymerisaten der WO 99/01967 wird das Farbmittel von der Polymermatrix eingekapselt. Diese farbmittelhaltigen Polymerisate zeigen daher ähnliche Vorteile wie ein unlösliches Pigment, d.h. das eingekapselte Farbmittel ist gegenüber äusseren Einflüssen, z.B. gegenüber photooxidativer Zerstörung oder gegenüber Ausbluten bei Lösungsmitteleinwirkung weitgehend inert. Gegenüber Pigmenten zeichnen sich die farbmittelhaltigen Polymerisate durch eine höhere Farbbrillanz aus. Ausserdem zeichnen sich farbmittelhaltige Polymerisate gegenüber Pigmenten häufig durch eine leichtere Handhabung und bessere Kompatiblität mit den anwendungsbezogenen Zubereitungen aus.

Die Herstellung der farbmittelhaltigen Polymerisate der WO 99/01967 erfolgt durch Polymerisation einer wässrigen farbmittelhaltigen Monomeremulsion. Zur Stabilisierung der Monomertröpfchen in der Emulsion wird in der Regel wenigstens ein anionischer Emulgator eingesetzt. Die zu diesem Zweck üblicherweise verwendeten anionischen Emulgatoren haben jedoch den Nachteil, dass sie zu Hautreizungen führen können. Dies ist insbesondere im Hinblick auf eine Verwendung dieser Farbmittel bedenklich. Der Ersatz dieser Emulgatoren ist daher wünschenswert.

Eigene Untersuchungen der Anmelderin haben jedoch gezeigt, dass bei Ersatz dieser anionischen Emulgatoren durch nichtionische Emulgatoren die Herstellung der farbmittelhaltigen Polymerisate aufgrund von Instabilitäten der zu polymerisierenden Monomeremulsionen nicht möglich ist. Ein Ersatz der anionischen Emulgatoren durch neutrale oder anionische Schutzkolloide führte ebenfalls nicht zum Erfolg.

Es wurde nun überraschenderweise gefunden, dass farbmittelhaltige wässrige Polymerdispersionen feinteiliger, farbmittelhaltiger Polymerisatteilchen auch in Abwesenheit anionischer Emulgatoren hergestellt werden können, wenn man die Polymerisation in Gegenwart eines oberflächenaktiven Stabilisatorsystems durchführt, das eine geeignete Menge wenigstens einer nichtionischen oberflächenaktiven Verbindung NO und eine geeignete Menge wenigstens eines amphiphilen Polymers PA, das 0,5 bis 10 mol/kg anionische funktionelle Gruppen aufweist, umfasst.

Demnach betrifft die vorliegende Erfindung zum einen farbmittelhaltige wässrige Polymerdispersionen, enthaltend:
a. farbmittelhaltiges Polymerisat in Form von Polymerisatteilchen PF mit einem gewichtsmittleren Teilchendurchmesser d₅₀ unterhalb 1000 nm, umfassend:
   i. eine aus ethylenisch ungesättigten Monomeren M aufgebaute Polymermatrix und
   ii. wenigstens ein in der Polymermatrix homogen verteiltes organisches Farbmittel F, ausgewählt unter Farbstoffen, UV-Absorbern und optischen Aufhellern, in einer Menge von 0,5 bis 50 Gew.-%, bezogen auf die Polymermatrix;
b. wenigstens eine nichtionische oberflächenaktive Verbindung NO in einer Menge von 0,1 bis 20 Gew.-% bezogen auf die Polymermatrix; und
c. wenigstens ein amphiphiles Polymer PA, das 0,5 bis 10 mol/kg anionische funktionelle Gruppen aufweist, in einer Menge von 1 bis 50 Gew.-%, bezogen auf die Polymermatrix.

Die Erfindung betrifft auch ein Verfahren zu deren Herstellung, die aus den farbmittelhaltigen wässrigen Polymerdispersionen erhältlichen Polymerpulver und kosmetische Mittel, die die erfindungsgemässen, farbmittelhaltigen wässrigen Polymerdispersionen und/oder die daraus hergestellten Pulver enthalten. Ein wichtiger Vorteil der erfindungsgemäßen Farbmittel ist, dass sie in Abwesenheit von anionischen Emulgatoren hergestellt werden können und daher keine anionischen Emulgatoren enthalten müssen, d.h. der Gehalt an anionischen Emulgatoren ist in der Regel < 0,1 Gew.-%, insbesondere < 0,05 Gew.-% und besonders bevorzugt < 0,01 Gew.-%, bezogen auf das farbstoffhaltige Polymerisat PF (Polymermatrix + eingekapseltes Farbmittel F).

Unter homogener Verteilung des organischen Farbmittels versteht man, dass das organische Farbmittel in der polymeren Matrix des farbmittelhaltigen Polymerisats molekulardispers verteilt, d.h. monomolekular gelöst oder in Form von bi- oder höhermolekularen Farbmittelaggregaten gelöst, vorliegt.

Der Begriff Farbmittel umfasst hier und im Folgenden chemische Verbindungen oder Salze chemischer Verbindungen sowie Charge-Transfer-Komplexe von chemischen Verbindungen, die ausgewählt sind unter Farbstoffen, optischen Aufhellern und UV-Absorbern. Diese Verbindungen weisen ein ausgedehntes π-Elektronensystem mit in der Regel wenigstens 8 π-Elektronen auf. Im Falle der Farbstoffe besitzen diese Verbindungen ein Absorptionsmaximum im Wellenlängenbereich von 400 bis 850 nm auf und rufen somit für das menschliche Auge einen Farbeindruck hervor (konventionelle Farbstoffe) und emittieren gegebenenfalls auch selber Licht im sichtbaren Bereich (Fluoreszenzfarbstoffe). Optische Aufheller besitzen ein oder mehrere Absorptionsmaxima im Bereich von 250 bis 400 nm und emittieren bei Bestrahlen mit UV-Licht eine Fluoreszenzstrahlung im sichtbaren Bereich. UV-Absorber absorbieren Licht der Wellenlänge < 400 nm und wandeln es in Wärmestrahlung um.

Unter C₁-C₂₀-Alkyl ist hier und im Folgenden sowohl lineares als auch verzweigtes Alkyl mit 1 bis 20 Kohlenstoffatomen zu verstehen. Beispiele hierfür sind Methyl, Ethyl, n-Propyl, i-Propyl, n-Butyl, 2-Butyl, Isobutyl, tert.-Butyl, n-Pentyl, Isopentyl, Neopentyl, tert.-Pentyl, Hexyl, 2-Methylpentyl, Heptyl, 1-Ethylpentyl, n-Octyl, 2-Ethylhexyl, Isooctyl, Nonyl, Isononyl, Decyl, Isodecyl, Undecyl, Dodecyl, Tridecyl, Isotridecyl, Tetradecyl, Pentadecyl, Hexadecyl, Heptadecyl, Octadecyl, Nonadecyl, Eicosyl.

Unter C₅-C₁₀-Cycloalkyl sind aliphatische Monocyclen wie Cyclopentyl, Cyclohexyl und Cycloheptyl sowie aliphatische Polycyclen wie Norbornyl, Adamantyl oder Decahydronaphthyl zu verstehen.

Erfindungswesentlich ist, dass sich das Farbmittel F in der polymeren Matrix des farbmittelhaltigen Polymerisats homogen verteilen lässt. Dies ist in der Regel dann gewährleistet, wenn das organische Farbmittel F, gegebenenfalls in Form eines Salzes, in den niedermolekularen Bestandteilen, welche die Polymermatrix bilden (Monomere M), eine zumindest begrenzte Löslichkeit aufweist. Vorzugsweise weist das organische Farbmittel F eine Löslichkeit auf, die größer ist als die geplante Einsatzmenge im Polymeren. Geeignete Farbmittel F weisen daher eine Löslichkeit ≥ 1 Gew.-%, insbesondere ≥ 2 Gew.-% insbesondere ≥ 5 Gew.-% und ganz besonders bevorzugt ≥ 10 Gew.-% in den Monomeren M auf.

Je nach Absorptionsstärke des Farbmittels F enthält das farbmittelhaltige Polymerisat PF in der Regel wenigstens 0,5 Gew.-%, bezogen auf das Gewicht der Polymermatrix, vorzugsweise 1 bis 40 Gew.-% und insbesondere 2 bis 30 Gew.-% wenigstens eines organischen Farbmittels F.

Beispiele für monomerlösliche, neutrale Farbstoffe sind die gemäß Colour-Index als Disperse-Farbstoffe und die als Solvent-Farbstoffe bezeichneten Verbindungen, die auch als Dispersionsfarbstoffe bezeichnet werden. Eine Zusammenstellung geeigneter Dispersionsfarbstoffe findet sich beispielsweise in Ullmanns Enzyklopädie der technischen Chemie, 4. Auflage, Bd. 10, S. 155-165 (siehe auch Bd. 7, S. 585ff - Anthrachinonfarbstoffe; Bd. 8, S. 244ff - Azofarbstoffe; Bd. 9, S. 313ff - Chinophthalonfarbstoffe). Auf diese Literaturstelle und die darin genannten Verbindungen wird hiermit ausdrücklich Bezug genommen.

Erfindungsgemäß geeignete Dispersionsfarbstoffe und Solvent-Farbstoffe umfassen verschiedenste Farbstoffklassen mit unterschiedlichen Chromophoren, beispielsweise Anthrachinonfarbstoffe, Monoazo- und Disazofarbstoffe, Chinophthalone, Methin- und Azamethinfarbstoffe, Naphthalimidfarbstoffe, Naphthochinonfarbstoffe und Nitrofarbstoffe. Beispiele für erfindungsgemäß geeignete Dispersionsfarbstoffe sind die Dispersionsfarbstoffe der folgenden Colour-Index Liste:
C. I. Disperse Yellow 1, 2, 3, 4, 5, 6, 7, 8, 9, 10, 11, 11:1, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119. 120, 121, 179, 180, 181, 182, 183, 184, 184:1, 200, 201, 202, 203, 204, 205, 206, 207, 208, 209, 210, 211, 212, 213, 214, 215, 216, 217, 218, 219, 220, 221, 222, 223, 224, 225, 226, 227, 228.
C. I. Disperse Orange 1, 2, 3, 3:3, 4, 5, 6, 7, 8, 9, 10, 11, 12, 13, 14, 15, 16, 17, 18,19, 20, 21, 22, 23, 24, 25, 25:1, 26, 27, 28, 29, 30, 31, 32, 33, 34, 35, 36, 37, 38, 39, 40, 41, 41:1, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 126, 127, 128, 129, 130, 131, 136, 137, 138, 139, 140, 141, 142, 143, 145, 146, 147, 148.
C. I. Disperse Red 1, 2, 3, 4, 5, 5:1, 6, 7, 8, 9, 10, 11, 12, 13, 14, 15, 16, 17, 18, 19, 20, 21, 22, 23, 24, 25, 26, 27, 28, 29, 30, 30:1, 31, 32, 33, 34, 35, 36, 38, 39, 40, 41, 43, 43:1, 46, 48, 50, 51, 52, 53, 54, 55, 55:1, 56, 58, 59, 60, 61, 63, 65, 66, 69, 70, 72, 73, 74, 75, 76, 77, 79, 80, 81, 82, 84, 85, 86, 86: 1, 87, 88, 89, 90, 91, 92, 93, 94, 96, 97, 98, 100, 102, 103, 104, 105, 106, 107, 108, 109, 110, 111, 112, 113, 115, 116, 117, 118, 120, 121, 122, 123, 125, 126, 127, 128, 129, 130, 131, 132, 133, 134, 135, 136, 137, 138, 139, 140, 141, 142, 143, 144, 145, 146, 147, 148, 149, 150, 151, 151:1, 152, 153, 154, 155, 156, 157, 158, 159, 160, 161, 162, 163, 164, 165, 166, 167, 167:1, 168, 169, 170, 171, 172, 173, 174, 175, 176, 177, 178, 179, 180, 181, 182, 183, 184, 185, 186, 187, 188, 189, 190, 190:1, 191, 191:1, 192, 193, 194, 195, 211, 223, 273, 274, 275, 276, 277, 278, 279, 280, 281, 302:1, 305, 306, 307, 308, 309, 310, 311, 312, 313, 314, 315, 316, 317, 318, 319, 320, 321, 322, 323, 324, 325, 326, 327, 328 329, 330, 331, 332, 333, 334, 335, 336, 338, 339, 340, 341, 342, 343, 344, 346, 347, 348, 349.
C. I. Disperse Violet 1, 2, 3, 4, 4:1, 5, 6, 7, 8, 9, 10, 11, 12, 13, 14, 15, 16, 17, 18, 19, 20, 21, 22, 23, 24, 25, 26, 27, 28, 29, 31, 33, 34, 35, 36, 37, 38, 39, 40, 41, 42, 43, 44, 45, 46, 47, 48, 49, 50, 51, 52, 53, 54, 55, 56, 57, 58, 59, 60, 81, 86, 87, 88, 89, 91, 92, 93, 94, 95, 96, 97.
C. I. Disperse Blue 1, 1:1, 2, 3, 3:1, 4, 5, 6, 7, 7:1, 8, 9, 10, 11, 12, 13, 13:1, 14, 15, 16, 17, 18, 19, 20, 21, 22, 23, 23:1, 24, 25, 26, 27, 28, 29, 30, 31, 32, 33, 34, 35, 36, 38, 39, 40, 42, 43, 44, 45, 47, 48, 49, 51, 52, 53, 54, 55, 56, 58, 60, 60:1, 61, 62, 63, 64, 64:1, 65, 66, 68, 70, 72, 73, 75, 76, 77, 79, 80, 81, 81:1, 82, 83, 84, 85, 86, 87, 88, 89, 90, 91, 92, 93, 94, 95, 96, 97, 98, 99, 100, 101, 102, 103, 104, 105, 106, 107, 108, 109, 111, 112, 113, 114, 115, 116, 117, 118, 119, 121, 122, 123, 124, 125, 126, 127, 128, 130, 131, 132, 133, 134, 136, 137, 138, 139, 140, 141, 142, 143, 144, 145, 146, 147, 148, 149, 150, 151, 152, 153, 154, 155, 156, 158, 159, 160, 161, 162, 163, 164, 165, 165:2, 166, 167, 168, 169, 170, 171, 172, 173, 174, 175, 195, 281, 282, 283, 283:1, 284, 285, 286, 287, 288, 289, 290, 291, 292, 293, 294, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 349.
C. I. Disperse Green 1, 2, 5, 6, 9.
C. I. Disperse Brown 1, 2, 3, 4, 4:1, 5, 7, 8, 9, 10, 11, 18, 19, 20, 21.
C. I. Disperse Black 1, 3, 4, 5, 6, 7, 8, 9, 10, 11, 12, 13, 14, 15, 20, 22, 24, 25, 26, 27, 28, 29, 29:1, 30, 31, 32, 33, 34, 36.

Beispiele für erfindungsgemäß geeignete Solvent-Farbstoffe sind die Verbindungen der folgenden Colour-Index Liste:
C. I. Solvent Yellow 2, 3, 7, 12, 13, 14, 16, 18, 19, 21, 25, 25:1, 27, 28, 29, 30, 33, 34, 36, 42, 43, 44, 47, 56, 62, 72, 73, 77, 79, 81, 82, 83, 83:1, 88, 89, 90, 93, 94, 96, 98, 104, 107, 114, 116, 117, 124, 130, 131, 133, 135, 141, 143, 144, 145, 146, 157, 160:1, 161, 162, 163, 167, 169, 172, 174, 175, 176, 179, 180, 181, 182, 183, 184, 185, 186, 187, 189, 190, 191.
C. I. Solvent Orange 1, 2, 3, 4, 5, 7, 11, 14, 20, 23, 25, 31A, 40:1, 41, 45, 54, 56, 58, 60, 62, 63, 70, 75, 77, 80, 81, 86, 99, 102, 103, 105, 106, 107, 108, 109, 110, 111, 112, 113.
C. I. Solvent Red 1, 2, 3, 4, 8, 16, 17, 18, 19, 23, 24, 25, 26, 27, 30, 33, 35, 41, 43, 45, 48, 49, 52, 68, 69, 72, 73, 83:1, 84:1, 89, 90, 90:1, 91, 92, 106, 109, 111, 118, 119, 122, 124, 125, 127, 130, 132, 135, 141, 143, 145, 146, 149, 150, 151, 155, 160, 161, 164, 164:1, 165, 166, 168, 169, 172, 175, 179, 180, 181, 182, 195, 196, 197, 198, 207, 208, 210, 212, 214, 215, 218, 222, 223, 225, 227, 229, 230, 233, 234, 235, 236, 238, 239, 240, 241, 242, 243, 244, 245, 247, 248.
C. I. Solvent Violet 2, 8, 9, 11, 13, 14, 21, 21:1, 26, 31, 36, 37, 38, 45, 46, 47, 48, 49, 50, 51, 55, 56, 57, 58, 59, 60, 61.
C. I. Solvent Blue 2, 3, 4, 5, 7, 18, 25, 26, 35, 36, 37, 38, 43, 44, 45, 48, 51, 58, 59, 59:1, 63, 64, 67, 68, 69, 70, 78, 79, 83, 94, 97, 98, 99, 100, 101, 102, 104, 105, 111, 112, 122, 124, 128, 129, 132, 136, 137, 138, 139, 143.
C. I. Solvent Green 1, 3, 4, 5, 7, 28, 29, 32, 33, 34, 35.
C. I. Solvent Brown 1, 3, 4, 5, 12, 20, 22, 28, 38, 41, 42, 43, 44, 52, 53, 59, 60, 61, 62, 63.
C. I. Solvent Black 3, 5, 5:2, 7, 13, 22, 22:1, 26, 27, 28, 29, 34, 35, 43, 45, 46, 48, 49, 50.

Erfindungsgemäß geeignet sind weiterhin monomerlösliche Derivate des Naphthalins, des Anthracens, des Perylens, des Terylens, des Quarterylens, sowie monomerlösliche Diketopyrrolopyrrolfarbstoffe, Perinonfarbstoffe, Cumarinfarbstoffe, Isoindolin- und Isoindolinonfarbstoffe, Porphyrinfarbstoffe, Phthalocyanin- und Naphthalocyaninfarbstoffe.

Geeignete monomerlösliche Cumarinfarbstoffe sind beispielsweise in der US-A 3 880 869 und der DE-A 44 24 817 beschrieben, auf die hiermit in vollem Umfang Bezug genommen wird.

Geeignete unpolare Perylenfarbstoffe sind beispielsweise solche, wie sie in der US-A 4 618 694, der DE-A 24 51 782, der US-A 379 934, der US-A 4 446 324, der EP-A 277 980, der EP-A 657 436 oder der WO 96/22332 beschrieben sind. Weitere geeignete unpolare Perylenfarbstoffe lassen sich beispielsweise der EP-A 73 007 entnehmen. Auf die genannten Druckschriften wird hiermit in vollem Umfang Bezug genommen. Beispiele für bevorzugte Perylenfarbstoffe sind die 6,12-Dicyanoperylen-1,7-dicarbonsäure-C₂-C₁₀-alkylester, die Bis-(N-C₁-C₁₀-alkyl)perylentetracarbonsäurediimide und die entsprechenden N-(Alkylphenyl)-Verbindungen, die unter den Lumogen®F-Marken im Handel erhältlich sind (BASF Aktiengesellschaft, Deutschland), z. B. Lumogen®F Rot 300, Lumogen®F Gelb 083 und Lumogen®F Orange 240.

Geeignete Naphthalinfarbstoffe umfassen unter anderem Naphthalin-1,8-dicarbonsäureimide, die am Imid-Stickstoff mit unsubstituiertem, linearem oder verzweigtem C₁-C₂₀-Alkyl oder Aryl substituiert sind, und die in der 4- und/oder der 5-Position des Naphthalinrings C₁-C₆-Alkoxy-Substituenten aufweisen können.

Geeignete Anthracenfarbstoffe umfassen unter anderem 9,10-Diphenylanthracen, 9,10-Bisphenylethinylanthracen, 1,8-Dichloro-9,10-bisphenylethinylanthracen. Beispiele für geeignete Anthracenfarbstoffe lassen sich beispielsweise Ullmann's Encyclopedia of Industrial Chemistry, 5th Edition, Vol. A2, S. 402 f. entnehmen.

Geeignete Porphyrinfarbstoffe umfassen beispielsweise Tetraphenylporphyrin und Octaethylporphyrin sowie deren Zink- oder Nikkelkomplexe.

Beispiele für geeignete Phthalocyaninfarbstoffe sind Metallophthalocyanine, insbesondere Kupferphthalocyanine, die an den Phenyleneinheiten des Chromophors löslichkeitsvermittelnde Alkylgruppen mit vorzugsweise 4 bis 20 C-Atomen aufweisen, wobei die Alkylreste direkt oder über eine funktionelle Gruppe, beispielsweise über eine Sulfonamidgruppe an den Chromophor gebunden sein können. Handelsüblich sind beispielsweise Tetra-C₄-C₁₀-alkylphthalocyanin-Komplexe, wie Tetra-tert-butylkupferphthalocyanin oder Tetra-n-octylkupferphthalocyanin, sowie Sulfonamide von ein oder mehrfach sulfonierten Metallophthalocyaninen mit C₁₀-C₂₀-Alkylaminen, z. B. das Tetrasulfonamid des vierfach sulfonierten Kupferphthalocyanins mit Stearylamin.

Als Farbmittel F sind auch ionische Farbstoffe geeignet. Diese Farbstoffe sind als solche in der Regel nicht in der Polymermatrix löslich, können jedoch durch Derivatisierung nach bekanntem Verfahren in eine öllösliche, d. h. eine in der Polymermatrix lösliche Form (= Farbstoff F) überführt werden. Bei üblichen kationischen Farbstoffen können beispielsweise die Anionen gegen solche Anionen, die langkettige Alkylreste aufweisen, ausgetauscht werden. Zu den Anionen mit langkettigen Alkylresten zählen beispielsweise die Anionen langkettiger Carbonsäuren mit 8 bis 22 C-Atomen, Mono- und Dialkylphosphate mit 4 bis 22 C-Atomen je Alkylrest, Alkylsulfonate mit 8 bis 22 C-Atomen, z. B. Dodecylsulfonat. Entsprechend können Farbstoffe mit basischen Gruppen, die in der wässrigen Phase üblicherweise protoniert vorliegen, mit den Säuren der vorgenannten Anionen umgesetzt werden, wobei öllösliche Salze der Farbstoffe erhalten werden. Analog können Farbstoffe mit sauren funktionellen Gruppen bzw. mit anionischen Gruppen, z. B. Sulfat- oder Carboxylat-Gruppen, mit langkettigen Aminen oder Ammoniumsalzen, die wenigstens einen langkettigen organischen Rest aufweisen, in eine monomerlösliche Form überführt werden. Geeignete langkettige Carbonsäuren, bzw. deren Salze leiten sich von Fettsäuren, wie Caprinsäure, Palmitinsäure, Stearinsäure, Ölsäure, Linolsäure und Linolensäure ab. Geeignete Amine sind beispielsweise primäre, linear oder verzweigtkettige Alkylamine mit 8 bis 22 C-Atomen im Alkylrest.

Bei diesen Farbstoffen handelt es sich in der Regel um Farbstoffe der vorgenannten Farbstoffklassen, beispielsweise um Mono- oder Bisazofarbstoffe, die jeweils mindestens eine Sulfonsäuregruppe aufweisen, um Sulfonsäuregruppen tragende Triarylmethanfarbstoffe, um Kupferphthalocyaninsulfonsäure, um sulfonsäuregruppenhaltige Chinolinfarbstoffe oder Stilbenfarbstoffe. Beispielhaft genannt seien die folgenden Nummern des Colour-Index:
Direct Yellow 4, 5, 11, 50, 127, 137, 147, 153;
Acid Orange 7, 8;
Direct Orange 15, 34, 102;
Direct Red 81, 239, 252-255;
Direct Violet 9, 51;
Acid Blue 9, 86;
Direct Blue 199, 218, 267, 273, 279, 281;
Acid Black 194, 208, 210, 221;
Direct Black 19, 161, 170 und 171.

Kationische bzw. basische Farbstoffe umfassen beispielsweise _{A}zound Bisazofarbstoffe mit Aminogruppen oder Ammoniumgruppen, Triarylmethanfarbstoffe, oder Aminfarbstoffe, Methin- und Azamethinfarbstoffe, beispielsweise Basic Red 1, Basic Red 14, Basic Blue 7, Basic Blue 11, Basic Blue 26, Basic Violet 1, Basic Violet 4, Basic Violet 10 etc. Weiterhin zählen zu den monomerlöslichen Farbstoffen F auch Komplexe aus basischen und sauren Farbstoffen bzw. Komplexe aus anionischen und kationischen Farbstoffen, beispielsweise der Komplex aus Chrysoidinbase und Metanilgelbsäure.

Erfindungsgemäß zählen zu den Farbmitteln F auch optische Aufheller. Geeignete optische Aufheller sind beispielsweise Verbindungen der Klassen der Bisstyrylbenzole, der Stilbene, der Benzoxazole, der Cumarine, der Pyrene und der Naphthaline. Es ist möglich, die oben genannten Aufheller alleine oder auch als Mischungen untereinander anzuwenden.

Bei den oben genannten optischen Aufhellern handelt es sich in der Regel um an sich bekannte und handelsübliche Produkte. Sie sind beispielsweise in Ullmann's Encyclopedia of Industrial Chemistry, 5. Auflage, Band A18, S. 156-161, beschrieben oder können nach den dort genannten Methoden erhalten werden.

Zu den organischen Farbmitteln F zählen auch UV-strahlenabsorbierende Verbindungen (UV-Absorber), die die absorbierte Strahlung strahlungslos deaktivieren. Derartige Verbindungen werden häufig in Sonnenschutzmitteln eingesetzt. Zu den UV-Absorbern zählen Derivate der p-Aminobenzoesäure, insbesondere deren Ester; Salicylate, substituierte Zimtsäureester (Cinnamate) wie Octyl-p-methoxycinnamat, Isopentyl-4-methoxycinnamat, Benzophenone, wie 4-Methoxy-2-hydroxybenzophenon-Sulfonsäure-Natriumsalz, Salicylate wie 4-Isopropylbenzylsalicylat, 4-Aminobenzoesäure und ihre Derivate wie ethoxilierter 4-Aminobenzoesäureethylester, 2-Ethylhexyl-4,4-dimethylaminobenzoat, Ester der 4,4-Diphenylbutadien-1,1-dicarbonsäure, z.B. deren Bis(2-ethylhexyl)ester, 2-Phenylbenzimidazol-4-sulfonsäure und deren Salze, Urocainsäure, deren Salz und deren Ester, Benzoxazole, Benzotriazole wie 2-(2H-Benzotriazol-2-yl)-4-methyl-6-(2-methyl-3-((1,1,3,3-tetramethyl-1-(trimethylsilyloxy)disiloxanyl)propyl)phenol, Benzylidencampher und seine Derivate, wie sie z. B. in der DE-A 3 836 630 genannt sind, z.B. 3-Benzylidencampher, 3-(4'-Methylbenzyliden)d-1-campher, weiterhin α-(2-Oxoborn-3-yliden)toluol-4-sulfonsäure und ihre Salze, N,N,N-Trimethyl-4-(2-oxoborn-3-ylidenmethyl)anilinium-methosulfat, Dibenzoylmethane wie 4-tert.-Butyl-4'-methoxydibenzoylmethan, 2,4,6-Triaryltriazin-Verbindungen wie 2,4,6-Tris-{N-[4-(2-ethylhex-1-yl)oxycarbonylphenyl]amino}-1,3,5-triazin, 4,4'-((6-(((tert.-Butyl)aminocarbonyl)phenylamino)-1,3,5-triazin-2,4-diyl)imino)bis(benzoesäure-2'-ethylhexylester), 2-Cyano-3,3-diphenylacrylsäureethylester und -2'-ethylhexylester, sowie 2-Phenylbenzimidazol-5-sulfonsäure und ihre Salze. Weitere erfindungsgemäss geeignete UV-Absorber findet man in Cosmetic legislation, Vol.1, Cosmetic Products, European Commission 1999, S. 64-66, auf die hiermit Bezug genommen wird.

Die in den erfindungsgemässen farbmittelhaltigen Polymerdispersionen enthaltenen nichtionischen oberflächenaktiven Verbindungen NO weisen naturgemäss wenigstens einen polaren, hydrophilen Molekülteil, z.B. einen OH-Gruppen aufweisenden Molekülteil, z.B. einen Sorbitan-, Pentaerythrit- oder Glyceryl-Rest, vorzugsweise eine Poly-C₂-C₃-alkylenethergruppe mit wenigstens 2 und bis zu 300 Alkylenoxid-Wiederholungseinheiten und wenigstens einen lipophilen Molekülteil, vorzugsweise einen aliphatischen oder alicyclischen Kohlenwasserstoffrest, der wenigstens 6 C-Atome aufweist, auf. Derartige Verbindungen sind aus dem Stand der Technik als nicht-ionische Emulgatoren bzw. Tenside oder nichtionische Detergenzien bekannt, beispielsweise aus Ullmanns Enzyclopedia of Industrial Chemistry, Vol.A9, 5^{th} ed., p.313 ff. Vorzugsweise weist die nichtionische oberflächenaktive Verbindung wenigstens eine Alkylgruppe mit 6 bis 32 C-Atomen und wenigstens eine Oligoethergruppe der allgemeinen Formel -[CH₂CH₂O]ₙ-H aufweist, worin n wenigstens 2 ist und vorzugsweise im Bereich von 3 bis 250 liegt. Hierbei ist zu berücksichtigen, dass der Wert n einen Mittelwert darstellt, der im Folgenden auch als Ethoxilierunggrad bezeichnet wird.

Beispiele für Verbindungen NO sind
- Ethoxylate linearer oder verzweigter Alkanole mit 8 bis 36 und insbesondere 10 bis 22 C-Atomen und einem Ethoxilierungsgrad von 3 bis 250, insbesondere 3 bis 50, z.B. ethoxilierter C_{13/15}-Oxoalkohol mit 3 bis 20 Ethlyenoxideinheiten, C_{12/14}-Fettalkohol mit 3 bis 20 Ethlyenoxideinheiten sowie polyethoxilierter Cetylalkohol.
- Ethoxylate von Mono-, Di- und Tri-C₄-C₁₂-Alkylphenolen mit einem Ethoxilierungsgrad von 3 bis 50.
- Ester von C₈-C₃₂-Fettsäuren wie Stearinsäure, Palmitinsäure, Cocosfettsäure, Talgfettsäure, Laurinsäure oder Behensäure mit Oligo- bzw. Polyethylenoxid, das beispielsweise einen Oligomerisationsgrad von 2 bis 100 aufweist,
- Ethoxylate von C₈-C₂₂-Mono- und Dialkylaminen wie Octylamin, Stearylamin, Di-n-octylamin und Di-n-stearylamin mit Ethoxilierungsgraden im Bereich von 3 bis 50;
- Ethoxylate von Amiden der vorgenannten C₈-C₃₂-Fettsäuren mit Ethoxilierungsgraden im Bereich von 3 bis 50;
- Fettsäureester von ethoxiliertem Glycerin oder ethoxiliertem Sorbitan, z.B. Polyethoxylate von hydriertem Ricinusöl, ethoxiliertes Sorbitanmonolaurat (Ethoxilierungsgrad von 3 bis 50, z.B. 20).

Die Verbindungen NO sind in den erfindungsgemässen Polymerdispersionen in einer Menge von vorzugsweise 0,2 bis 10 und insbesondere von 0,3 bis 5 Gew.-%, bezogen auf die Polymermatrix des _{P}olymerisats PF enthalten.

Zur stabiliserung der Polymerisatteilchen enthält die erfindungsgemässe Polymerdispersion in einer Menge von 1 bis 50 Gew.-%, vorzugsweise 2 bis 30 Gew.-% und insbesondere 5 bis 25 Gew.-%, bezogen auf die Polymermatrix, wenigstens ein amphiphiles Polymer PA, das 0,5 bis 10 mol/kg und vorzugsweise 1 bis 8 mol/kg Polymer PA, anionische funktionelle Gruppen aufweist.

Anionische Gruppen leiten sich naturgemäss von Säuregruppen ab, aus denen sie durch Deprotonierung entstehen. Zu den anionischen Gruppen zählen beispielsweise Carboxylat-, Sulfonat-, Sulfat-, Phosphonat- und Phosphatgruppen. Bevorzugte Polymere PA weisen als anionische Gruppen Carboxylatgruppen auf. Als Gegenionen kommen sowohl Alkalimetall-, z.B. Natrium- oder Kaliumionen als auch Ammoniumionen in Betracht. Im Hinblick auf die Verwendung der erfindungsgemässen Polymerisatdispersionen in kosmetischen Mitteln sind als Gegenionen insbesondere NH₄⁺ sowie die Ammoniumionen hautverträglicher organischer Amine, insbesondere von Aminoalkoholen, wie 2-Amino-2-methylpropan-1-ol, Monoethanolamin, Diethanolamin, Triethanolamin, Triisopropanolamin, Tri[(2-hydroxy)-1-propyl]amin, 2-Amino-2-methylpropan-1,3-diol, 2-Amino-2-(hydroxymethyl)propan-1,3-diol und von Aminosäuren, wie Lysin, von Interesse.

Selbstverständlich müssen nicht alle der Säuregruppen im Polymer PA in neutralisierter Form vorliegen. In der Regel reicht ein Neutralisationsgrad von 50 % aller im Polymer vorhandenen Säuregruppen aus. Insbesondere beträgt der Neutralisationsgrad 80 bis 100 %.

Neben den polaren anionischen Gruppen weist das amphiphile Polymer naturgemäss auch hydrophile Atomgruppen, vorzugsweise in Form von C₁-C₃₂-Alkylgruppen auf, die direkt oder über eine Zwischenglied, beispielsweise über ein Sauerstoffatom, eine Polyethergruppe, eine Carbonyl-, Carbonyloxy- oder Carbonylamino-Gruppe an das Rückgrat des Polymeren PA gebunden sein können.

In den Polymeren PA kann ein Teil, vorzugsweise nicht mehr als 50 Mol-%, z.B. 1 bis 50 Mol-%, insbesondere 5 bis 25 Mol-% der Carboxylgruppen durch eine oberflächenaktive Verbindung mit Oligoethergruppe der allgemeinen Formel -[CH₂CH₂O]ₙ-H, worin n wenigstens 2 ist, z.B. mit einer der vorstehend beschriebenen Verbindungen NO, verestert sein.

Das gewichtsmittlere Molekulargewicht der Polymere PA liegt im Unterschied zu den anionischen Emulgatoren in der Regel oberhalb 1000 Dalton und insbesondere oberhalb 2000 Dalton. Vorzugsweise wird das Gewichtsmittlere Molekulargewicht einen Wert von 100000 Dalton, insbesondere von 70000 Dalton, nicht überschreiten.

Bevorzugt werden Polymere PA, die eine Kohlenstoffpolymerkette aufweisen und somit aus ethylenisch ungesättigten Monomeren M' aufgebaut sind.

In bevorzugten Polymeren PA umfassen die Monomere M':
- 5 bis 70 Mol-%, insbesondere 10 bis 60 mol-% wenigstens eines Monomers A, ausgewählt unter monoethlyenisch ungesättigten Mono- und Dicarbonsäuren mit 3 bis 8 C-Atomen;
- 30 bis 95 Mol-%, insbesondere 40 bis 90 Mol-% wenigstens eines begrenzt wasserlöslichen oder wasserunlöslichen Monomeres B und gegebenenfalls
- bis 30 Mol-% vorzugsweise bis 20 Mol-% eines von den Monomeren A und B verschiedenen Monomers C, jeweils bezogen auf die Summe der Monomere A, B und C.

Beispiele für Monomere A sind Acrylsäure, Methacrylsäure, Crotonsäure, Vinylessigsäure, 2-Ethylacrylsäure, 2-Acryloxyessigsäure, 2-Acrylamidoessigsäure, Maleinsäure, Maleinsäure-mono-C₁-C₄-alkylester wie Maleinsäuremonomethyl- und -monobutylester, Fumarsäure, Fumarsäure-mono-C₁-C₄-alkylester wie Fumarsäuremonomethylund -monobutylester, Itaconsäure und 2-Methylmaleinsäure.

Monomere B mit begrenzter Wasserlöslichkeit sind solche, die eine Wasserlöslichkeit von bis zu 80 g/l (bei 25°C und 1 bar) aufweisen. Sie bestimmen den hydrophoben Charakter der Polymere PA. In der Regel weisen derartige Monomere wenigstens eine C₁-C₂₀-Alkylgruppe auf. Beispiele für geeignete Monomere B sind:
- Vinyl- und Allylester aliphatischer Monocarbonsäuren mit 2 bis 20 C-Atomen wie Vinylacetat, Vinylpropionat, Vinylpivalat, Vinylversatat, Vinyllaurat und Vinylstearat;
- C₁-C₂₀-Alkyl- und C₅-C₁₀-Cycloalkylester der vorgenannten ethylenisch ungesättigten Mono- und Dicarbonsäuren, insbesondere der Acrylsäure und der Methacrylsäure. Bevorzugte Ester sind Methylmethacrylat, Ethylmethacrylat, n-Butylmethacrylat, tert.-Butylmethacrylat, Isobutylmethacrylat, n-Hexylmethacrylat, Cyclohexylmethacrylat, 2-Ethylhexylmethacrylat, Methylacrylat, Ethylacrylat, n-Butylacrylat, tert.-Butylacrylat, Isobutylacrylat, Cyclohexylacrylat und 2-Ethylhexylacrylat;
- Mono- und Di-C₁-C₂₀-alkylamide der vorgenannten ethylenisch ungesättigten Mono- und Dicarbonsäuren, insbesondere der Acrylsäure und der Methacrylsäure, z.B. N-tert.-Butylacrylamid und N-tert.-Butylmethacrylamid;
- C₃-C₂₀-Olefine wie Propen, 1-Buten, Isobuten, 2-Methylbuten, 1-Penten, 2-Methylpenten, 1-Hexen, 2-Methylhexen, 1-Octen, Isoocten, 2,4,4-Trimethylpenten (Diisobuten).
- vinylaromatische Monomere wie Styrol, α-Methylstyrol, Vinyltoluol und p-tert.-Butylstyrol.

Als Monomere C kommen vorzugsweise monoethylenisch ungesättigte Monomere in Betracht. Geeignet sind insbesondere neutrale Monomere C, die eine Wasserlöslichkeit oberhalb 80 g/l (bei 25°C und 1 bar) aufweisen. Beispiele für derartige Monomere sind die Amide der vorgenannten ethylenisch ungesättigten Monocarbonsäuren wie Acrylamid und Methacrylamid, N-Vinyllactame wie N-Vinylpyrrolidon und N-Vinylcaprolactam, Hydroxyalkylester der vorgenannten monoethylenisch ungesättigten Carbonsäuren wie Hydroxyethylacrylat, Hydroxypropylacrylat, Hydroxybutylacrylat, Hydroxyethylmethacrylat, Hydroxypropylmethacrylat, Hydroxybutylmethacrylat und die Ester der Acrylsäure oder der Methacrylsäure mit Oligoalkylenoxiden wie Oligoethylenoxid oder Oligopropylenoxid mit Oligomerisationsgraden im Bereich von 2 bis 200.

Der hier und im Folgenden verwendete Begriff Copolymer ist nicht auf Copolymere zweier verschiendener Monomere beschränkt sondern umfasst auch Polymere die aus drei oder mehr verschiedenen Monomeren aufgebaut sind.

Beispiele für bevorzugte Klassen der Polymere PA sind:
- Copolymere aus Maleinsäure als Monomer A und C₃-C₂₀-Olefinen als Monomer B, worin 1 bis 50 Mol-% und vorzugsweise 5 bis 30 Mol-% der Carboxylgruppen durch eine oberflächenaktive Verbindung mit Oligoethergruppe der allgemeinen Formel - [CH₂CH₂O]ₙ-H, worin n wenigstens 2 ist, verestert sein können. Derartige Polymere sind beispielsweise aus der EP-A 116 930 und der EP-A 367 049 bekannt, auf die hiermit Bezug genommen wird. Hierunter sind insbesondere Copolymere aus Methacrylsäure und Diisobuten im Molverhältnis 1:1 zu nennen, worin 1 bis 50 Mol-% und insbesondere 5 bis 30 Mol-% der Carbonsäuregruppen mit einem ethoxylierten C₁₀-C₂₂-Alkanol verestert sind, das einen Ethoxylierungsgrad von 3 bis 50 aufweist;
- Copolymere monoethylenisch ungesättigter C₃-C₈-Monocarbonsäuren als Monomere A mit Vinylestern aliphatischer C₂-C₂₀-Carbonsäuren als Monomere B, z.B. Copolymere aus Vinylacetat mit Crotonsäure im Molverhältnis 0,5:9,5 bis 2:8;
- Copolymere aus Monomeren A mit Monomeren B, ausgewählt unter den C₁-C₂₀-Alkylestern monoethylenisch ungesättigter Monocarbonsäuren und den N-C₁-C₂₀-Alkyl- und N,N-(Di-C₁-C₂₀-alkyl)amiden monoethylenisch ungesättigter C₃-C₈-Monocarbonsäuren. Hier sind beispielsweise Terpolymere aus N-tert.-Butylacrylamid, Ethylacrylat und Acrylsäure oder aus tert.-Butylacrylat, Ethylacrylat und Methacrylsäure mit Säurezahlen im Bereich von 60 bis 200 mg KOH/g Polymer zu nennen; entsprechend einem Carboxylgruppengehalt von 1 bis 3,5 mol/kg.

Die farbmittelhaltigen Polymerteilchen PF umfassen eine Polymermatrix, die erfindungsgemäss aus ethylenisch ungesättigten Monomeren M aufgebaut ist. Die gewichtsmittlere Teilchengrösse (Teilchendurchmesser) der Polymerteilchen liegt vorzugsweise im Bereich von 50 bis 500 nm und insbesondere im Bereich von 100 bis 400 nm. Die mittlere Teilchengröße entspricht dem Volumenmittel, welches in bekannter Weise durch quasielastische Lichtstreuung einer verdünnten wässrigen Dispersion der Polymerteilchen (berechnet durch unimodale Analyse der Autokorrelationsfunktion) ermittelt werden kann. Die Messungen werden in der Regel an 0,1 gew.-%igen Proben bei Normalbedingungen (1 bar, 25 °C) vorgenommen. Die Messung kann beispielsweise mit einem Coulter N4 Plus Particle Analyzer der Fa. Coulter Scientific Instruments vorgenommen werden.

Die polymere Matrix umfasst in der Regel wenigstens ein hydrophobes Monomer M1 mit einer Wasserlöslichkeit im Bereich von 0,01 g/l bis 80 g/l, insbesondere 0,1 bis 50 g/l (bei 25 °C und 1 bar). Die Monomere A machen in der Regel wenigstens 70 Gew.-% und insbesondere wenigstens 80 Gew.-%, z.B. 70 bis 99,9 Gew.-% und insbesondere 80 bis 99 Gew.-%, bezogen auf das Gesamtgewicht der Monomere M aus.

Die Monomere M1 sind vorzugsweise ausgewählt unter:
- C₁-C₂₀-Alkyl- und C₅-C₁₀-Cycloalkylestern der vorgenannten ethylenisch ungesättigten Mono- und Dicarbonsäuren, insbesondere der Acrylsäure und der Methacrylsäure. Bevorzugte Ester sind Methylmethacrylat, Ethylmethacrylat, n-Butylmethacrylat, tert.-Butylmethacrylat, Isobutylmethacrylat, n-Hexylmethacrylat, Cyclohexylmethacrylat, 2-Ethylhexylmethacrylat, Methylacrylat, Ethylacrylat; n-Butylacrylat, tert.-Butylacrylat, Isobutylacrylat, Cyclohexylacrylat und 2-Ethylhexylacrylat;
- Vinylestern von C₁-C₈-Monocarbonsäuren. Beispiele für Vinylester sind Vinylacetat, Vinylpropionat, Vinylbutyrat und Vinylhexanoat;
- vinylaromatischen Monomeren wie vorstehend genannt, insbesondere Styrol
- C₂-C₆-Olefinen, wie Ethylen, Propen, 1-Buten, 2-Buten und Isobuten.

Es hat sich als vorteilhaft erwiesen, wenn die Monomere M neben den Monomeren M1 auch vernetzende Monomere M2 umfassen, die wenigstens zwei nicht konjugierte ethylenisch ungesättigte Doppelbindungen aufweisen. Durch die Monomere M2 wird eine bessere Einbindung des Farbmittels in die Polymermatrix erreicht. Die Monomere M2 machen in der Regel 0,1 bis 30 Gew.-%, vorzugsweise 0,5 bis 20 Gew.-%, insbesondere 1 bis 10 Gew.-% der Monomere M aus.

Geeignete Monomere M2 umfassen z. B. die Vinyl-, Allyl- und Methallylester der oben genannten ethylenisch ungesättigten Carbonsäuren ebenso wie die Ester dieser Säuren mit Tricyclodecenylalkohol, insbesondere die Ester der Methacrylsäure und der Acrylsäure, die Ester der oben genannten ethylenisch ungesättigten Carbonsäuren mit mehrwertigen Alkoholen, wie Ethylenglykoldiacrylat, Ethylenglykoldimethacrylat, Butandioldiacrylat, Butandioldimethacrylat, Hexandioldiacrylat, Hexandioldimethacrylat, Triethylenglykoldiacrylat, Triethylenglykoltrimethacrylat, Tris(hydroxymethyl)ethantriacrylat und -trimethacrylat, Pentaerythrittriacrylat und -trimethacrylat, ferner die Allyl- und Methallylester von polyfunktionellen Carbonsäuren, wie Diallylmaleat, Diallylfumarat, Diallylphthalat. Typische Monomere B1 sind auch Verbindungen, wie Divinylbenzol, Divinylharnstoff, Diallylharnstoff, Triallylcyanurat, N,N'-Divinyl- und N,N'-Diallylimidazolidin-2-on, sowie Methylenbisacrylamid und Methylenbismethacrylamid.

Im Hinblick auf die Herstellbarkeit der erfindungsgemäßen farbmittelhaltigen wässrigen Polymerdispersionen der Polymerisate PF hat es sich außerdem als vorteilhaft erwiesen, wenn bei der Polymerisation der Monomere M besonders hydrophobe Monomere mit einer Wasserlöslichkeit < 0,01 g/l (bei 25 °C und 1 bar) zugegen sind (Monomere M3). Monomere M3 werden, sofern erwünscht, in einer Menge von 0,1 bis 20 Gew.-%, insbesondere in einer Menge von 1 bis 10 Gew.-%, bezogen auf die Monomere M verwendet.

Beispiele für Monomere M3, die eine wie vorstehend geforderte geringe Wasserlöslichkeit aufweisen, sind 2- und 4-n-Butylstyrol, p-tert.-Butylstyrol, Ester aus 3 bis 6 C-Atome aufweisenden α,β-monoethylenisch ungesättigten Carbonsäuren und ≥ 12 C-Atome (in der Regel bis zu 30 C-Atome) aufweisenden Alkanolen wie z. B. Laurylacrylat und Stearylacrylat. Zu den Monomeren M3 zählen auch Ester aus Vinylalkohol oder Allylalkohol und ≥ 9 C-Atome (in der Regel bis zu 30 C-Atome) aufweisenden Alkancarbonsäuren, wie z. B. Vinylnonanoat, Vinyldecanoat, Vinyllaurat und Vinylstearat, sowie im Handel befindliche Monomere VEOVA® 9-11 (VEOVA X ist ein Handelsname der Firma Shell und steht für Vinylester von Carbonsäuren, die auch als Versatic® X-Säuren bezeichnet werden). Zu den Monomeren M3 zählen Makromonomere wie Oligopropenacrylat (ganz allgemein sind Makromonomere polymere oder oligomere Verbindungen, die wenigstens eine, meist endständige, ethylenisch ungesättigte Doppelbindung aufweisen; ihr relatives zahlenmittleres Molekulargewicht sollte für eine Verwendbarkeit als geringst wasserlösliches Monomeres M3 vorzugsweise nicht mehr als 100000 betragen; in der Regel wird dieses relative zahlenmittlere Molekulargewicht 1000 bis 50000 bzw. 2000 bis 50000 betragen; Makromonomere sind dem Fachmann bekannt; ihre Herstellung ist beispielsweise in Makromol. Chem. 223 (1994) S. 29 bis 46 beschrieben). Ganz allgemein kommen als geringst wasserlösliche Monomere M3 alle diejenigen in Betracht, deren molare Löslichkeit bei 25 °C und 1 atm in Wasser gleich oder geringer als die entsprechende Löslichkeit von Laurylacrylat ist. Solche Monomeren M3 sind z. B. auch das Methacryloyl-Polybutylacrylat AB-6 und das Methacryloyl-Polystyrol A5-6 der Fa. Toa Gosei Kagaku KK (JP), die beide ein zahlenmittleres relatives Molekulargewicht von 6000 aufweisen. Aber auch Polyol 130 und Polyol 110 der Hüls AG (stereospezifisches, niedrigviskoses Polybutadien (75 % 1,4-cis, 24 % 1,4-trans, 1 % vinyl), dessen dynamische Viskosität bei 20 °C 3000 mPas beträgt) bilden als Makromonomere mit geringer Wasserlöslichkeit einsetzbare Monomere M3. Anstelle der Polymere M3 kann man auch nichtpolymerisierbare Verbindungen mit einer Wasserlöslichkeit < 0,01 g/l bei der Herstellung der erfindungsgemäßen, farbmittelhaltigen, wässrigen Polymerisatdispersionen einsetzen.

Die Monomere M können weiterhin in einer Menge von bis zu 30 Gew.-%, vorzugsweise nicht mehr als 20 Gew.-% und insbesondere nicht mehr als 10 Gew.-% Monomere M4 umfassen, die von den vorgenannten Monomeren M1, M2 und M3 verschieden sind. Hierzu zählen Monomere, deren Homopolymerisate eine erhöhte Wasserlöslichkeit (d. h. > 80 g/l bei 25 °C) aufweisen. Derartige Monomere M4 dienen als modifizierende Monomere. Zu den Monomeren M4 zählen sowohl monoethylenisch ungesättigte Monomere mit wenigstens einer Säuregruppe, z. B. einer COOH-, SO₃H- oder einer PO₃H₂-Gruppe, die auch in Salzform vorliegen kann (im Folgenden als anionische Monomere bezeichnet) und monoethylenisch ungesättigte, neutrale Monomere.

Beispiele für monoethylenisch ungesättigte anionische Monomere D (Monomere DA) sind die vorgenannten monoethylenisch ungesättigten Mono- und Dicarbonsäuren, insbesondere Acrylsäure und Methacrylsäure, monoethylenisch ungesättigte Sulfonsäuren und Phosphonsäuren, z. B. Vinylsulfonsäure, Allylsulfonsäure, Methallylsulfonsäure, Styrolsulfonsäure, Vinylnaphthalinsulfonsäure und (Meth)acrylamido-2-methylpropansulfonsäure, weiterhin Vinylphosphonsäure, Allylphosphonsäure, Methallylphosphonsäure, Styrolphosphonsäure, und (Meth)acrylamido-2-methylpropanphosphonsäure, sowie deren wasserlösliche Salze, z. B. deren Alkalimetallsalze oder deren Ammoniumsalze, insbesondere deren Natriumsalze. Beispiele für neutrale Monomere M4 sind insbesondere die Amide monoethylenisch ungesättigter Mono- und Dicarbonsäuren, wie Acrylamid, Methacrylamid, weiterhin N-Vinyllactame mit 3 bis 8 C-Atomen, wie N-Vinylpyrrolidon und N-Vinylcaprolactam. Zu den Monomeren M4 zählen auch Acrylnitril und Methacrylnitril, deren Einsatz häufig zu einer besseren Löslichkeit des Farbmittels in den Monomeren M und damit zu einer besseren Verteilung des Farbmittels in der polymeren Matrix führen. Acrylnitril und Methacrylnitril werden, sofern erwünscht, häufig in Mengen bis zu 30 Gew.-%, z.B. in Mengen von 0,5 bis 30 Gew.-%, bezogen auf das Gesamtgewicht der Monomere M, bei der Herstellung der farbmittelhaltigen Polymerisate PF eingesetzt.

Unter den vorgenannten Polymerisaten werden für die erfindungsgemäße Verwendung insbesondere solche farbmittelhaltigen Polymerisate PF bevorzugt, worin 50 Gew.-% der Monomere M1 unter solchen Monomeren ausgewählt sind, deren Homopolymerisate eine Glasübergangstemperatur > 40 °C aufweisen (Monomere M1a) und weniger als 50 Gew.-%, insbesondere weniger als 30 Gew.-% und besonders bevorzugt weniger als 10 Gew.-% solcher Monomere M1b, deren Homopolymerisate eine Glasübergangstemperatur < 40 °C aufweisen. Beispiele für besonders bevorzugte Monomere Mla sind Methylacrylat, Methylmethacrylat, Ethylmethacrylat, tert.-Butylacrylat, Vinylacetat, Styrol, Vinyltoluol und Methacrylnitril.

In einer ganz besonders bevorzugten Ausführungsform der erfindungsgemäßen, farbmittelhaltigen Polymerisate ist die polymere Matrix aufgebaut aus:
- 80 bis 99 Gew.-% Monomeren M1, insbesondere Monomeren M1a, besonders bevorzugt aus Methylacrylat, Styrol, Methylmethacrylat oder deren Mischungen, wobei bis zu 30 Gew.-% der Monomere M1 durch Acrylnitril ersetzt sein können,
- 1 bis 20 Gew.-%, insbesondere 2 bis 10 Gew.-%, Monomeren M2, z. B. Divinylbenzol oder 1,4-Butandioldiacrylat,
- 0 bis 20 Gew.-%, z. B. 1 bis 20 Gew.-%, Monomeren M3, z. B. Laurylacrylat oder Stearylacrylat, und
- 0 bis 20 Gew.-%, z. B. 1 bis 20 Gew.-%, Monomeren M4, z. B. Acrylsäure, Methacrylsäure, Acrylamid, Methacrylamid, Acrylamido-2-methylpropansulfonat-Natriumsalz.

Die Herstellung der erfindungsgemässen wässrigen Dispersionen farbmittelhaltiger Polymerisate PF erfolgt durch radikalische wässrige Emulsionspolymerisation einer Öl-in-Wasser-Emulsion der Monomere M, deren Monomertröpfchen (= Emulsionströpfchen) das Farbmittel in gelöster Form enthalten und die im Unterschied zu konventionellen Öl-in-Wasser-Emulsionen einen Tröpfchendurchmesser < 1000 nm aufweisen. Derartige feinteilige Monomeremulsionen werden auch als "Mini-Emulsionen" bezeichnet (vgl. P.L. Tang, E.D. Sudol, C.A. Silebi und M.S. El-Aasser in Journal of Applied Polymer Science, Vol. 43, S. 1059 - 1066 [1991]). Bevorzugt weisen die Tröpfchen einen mittleren Durchmesser d_{z} von ≤ 500 nm und insbesondere ≤ 400 nm auf. In der Regel wird d_{z} wenigstens 40 nm und vorzugsweise wenigstens 100 nm betragen. Die Tröpfchengröße der Öl-in-Wasser-Emulsion der Monomere wird ähnlich wie die Teilchengröße der Polymerisatteilchen des Polymerisats PF durch quasielastische, dynamische Lichtstreuung ermittelt. Vorzugsweise weisen die Tröpfchen in der farbmittelhaltigen Monomeremulsion eine weitgehend einheitliche Größe auf, d. h. der Quotient (d₉₀-d₁₀)/d₅₀ hat einen Wert ≤ 1, vorzugsweise ≤ 0,5, insbesondere ≤ 0,25. Hierin steht dₙ für den Teilchendurchmesser, den n Gew.-% der Emulsionströpfchen unterschreiten.

Zur Herstellung der farbmittelhaltigen Monomer-Miniemulsionen löst man zunächst das Farbmittel F in den zu polymerisierenden Monomeren M. Die so erhaltene Farbmittellösung wird dann nach üblichen Methoden, beispielsweise durch Einrühren oder Dispergieren in einer wässrigen Lösung einer oberflächenaktiven Substanz, in eine Öl-in-Wasser-Emulsion überführt. Die oberflächenaktive Substanz ist erfindungsgemäss ausgewählt unter den oben definierten nichtionischen Verbindungen NO und dem amphiphilen Polymer PA. Zur Emulgierung wird entweder ein Teil oder die gesamte zur Herstellung der erfindungsgemässen Polymerdispersion benötigte Menge an Verbindung NO und amphiphilem Polymer PA eingesetzt. Beispielsweise kann man die Emulgierung in Gegenwart von Verbindung NO als alleiniger oberflächenaktiver Substanz durchführen und anschliessend nach der Emulgierung das amphiphile Polymer PA zugeben und umgekehrt.

Die so erhaltenen wässrigen Emulsionen weisen in der Regel mittlere Tröpfchengrößen d_{z} oberhalb von 1000 nm auf. Diese konventionellen "Makroemulsionen" werden dann durch Homogenisieren in Monomeremulsionen mit Tröpfchengrößen ≤ 1000 und vorzugsweise ≤ 500 nm überführt. Die Polymerisation von Monomeremulsionen mit Tröpfchengrößen ≤ 500 nm führt zu besonders hochwertigen farbmittelhaltigen Polymerisaten PF.

Die Homogenisierung erfolgt bevorzugt durch Anwendung von Ultraschall (z. B. Branson Sonifier II 450). Für die Homogenisierung mittels Ultraschall sind beispielsweise die in der GB 22 50 930 A und der US 5,108,654 beschriebenen Vorrichtungen geeignet. Die Anwendung von Ultraschall hat sich zur Herstellung der farbmittelhaltigen Mini-Emulsionen besonders bewährt und führt in der Regel zu besonders hochwertigen farbmittelhaltigen Polymerisaten PF.

Anschliessend führt man die Polymerisation der Miniemulsion in Gegenwart eines die radikalische Polymerisation der Monomere M auslösenden Polymerisationsinitiators durch, wobei erfindungsgemäss die Polymerisation in Gegenwart von 0,1 bis 20 Gew.-% wenigstens einer nichtionischen oberflächenaktiven Verbindung NO und 1 bis 50 Gew.-%, jeweils bezogen auf die Monomere M, wenigstens eines amphiphilen Polymers PA erfolgt.

Dabei kann man so vorgehen, dass man die farbmittelhaltige Emulsion, vorzugsweise in Form einer Mini-Emulsion im Reaktor vorlegt und hierzu den Polymerisationsinitiator unter Polymerisationsbedingungen in einer Portion oder in mehreren Portionen oder kontinuierlich nach Maßgabe seines Verbrauchs zugibt. Auch kann man zunächst einen Teil oder die Gesamtmenge des Polymerisationsinitiators zu der Menge Emulsion geben und anschließend auf Polymerisationstemperatur erwärmen.

Es ist auch möglich, einen Teil oder die Gesamtmenge der wässrigen, farbmittelhaltigen Monomeremulsion, vorzugsweise in Form einer Mini-Emulsion, dem Polymerisationsgefäß unter Polymerisationsbedingungen nach Maßgabe des Fortschreitens der Umsetzung zuzugeben. Vorzugsweise führt man dabei den Polymerisationsinitiator zumindest teilweise parallel zur Zugabe der Monomeremulsion dem Polymerisationsgefäß zu.

Als radikalische Polymerisationsinitiatoren kommen prinzipiell alle diejenigen in Betracht, die in der Lage sind, eine radikalische Polymerisation auszulösen. Es handelt sich hierbei sowohl um Peroxide, Hydroperoxide als auch um Azoverbindungen. Die radikalischen Polymerisationsinitiatoren können sowohl wasserlöslich sein als auch öllöslich, d. h. in den Monomeren löslich, sein.

Beispiele für wasserlösliche Initiatoren sind Peroxodischwefelsäure und ihre Ammonium- und Alkalimetallsalze, Wasserstoffperoxid oder niedermolekulare Hydroperoxide, wie tert.-Butylhydroperoxid, oder salzartige Azoverbindungen, z. B. 2,2'-Azobis-2-amidinopropan-dihydrohalogenid.

Beispiele für öllösliche Polymerisationsinitiatoren sind C₄-C₁₂-Peroxocarbonsäuren sowie ihre Ester, z. B. Peroctoate und Perbenzoate, wie tert.-Butylperoctoat und tert.-Butylperbenzoat sowie Diacylperoxide, wie Dibenzoylperoxid.

Die vorgenannten, wasserlöslichen peroxidischen Polymerisationsinitiatoren können auch mit einem Reduktionsmittel und gegebenenfalls mit einer im wässrigen Medium löslichen Metallverbindung kombiniert werden (sogenannte Redox-Initiatorsysteme). Diese sind dem Fachmann hinreichend bekannt. Wegen weiterer Details wird auf die WO 99/40123 verwiesen.

Die Menge an eingesetztem Initiator liegt in der Regel im Bereich von 0,1 bis 10 Gew.-%, vorzugsweise 0,2 bis 8 Gew.-% und häufig im Bereich von 0,3 bis 5 Gew.-%.

Bei der Herstellung der wässrigen Dispersionen der farbmittelhaltigen Polymerisate PF richten sich die Polymerisationstemperaturen in ersten Linie nach dem jeweils eingesetzten Initiatorsystem in bekannter Weise. Üblicherweise liegen die Polymerisationstemperaturen im Bereich von 0 bis 95 °C, vorzugsweise im Bereich von 30 bis 90 °C. Bei Anwendung von erhöhtem Druck kann die Polymerisationstemperatur auch bis zu 120 °C betragen. Üblicherweise wird bei Normaldruck (1 Atmosphären) polymerisiert.

Das erfindungsgemässe Verfahren liefert die erfindungsgemässen farbmittelhaitigen wässrigen Polymerdispersionen ohne nennenswerte Koagulatbildung, ohne dass zur Stabilisierung der Polymerteilchen und der Monomertröpfchen anionische Emulgatoren erforderlich sind. Die erfindungsgemässen farbmittelhaltigen wässrigen Polymerdispersionen enthalten somit als oberflächenaktive Substanz lediglich die vorstehend beschriebenen Verbindungen NO und wenigstens ein amphiphiles Polymer PA. Ausserdem zeichnen sich die erfindungsgemässen Polymerdispersionen durch eine gute Lagerstabilität aus.

Die vorliegende Erfindung betrifft ausserdem farbmittelhaltige Polymerpulver, die durch Verdampfen der flüchtigen Bestandteile der erfindungsgemässen, wässrigen farbmittelhaltigen Polymerdispersion erhältlich sind. Das Verdampfen der flüchtigen Bestandteile erfolgt in Analogie zu bekannten Verfahren der Pulverherstellung aus wässrigen Polymerdipsersionen.

Überraschenderweise gelingt das Verdampfen der flüchtigen Bestandteile ohne den üblicherweise erforderlichen Zusatz von Sprühhilfsmitteln. Anders als bei konventionellen Polymerdispersionen zeigen die erfindungsgemässen Polymerdispersionen beim Verdampfen der flüchtigen Bestandteile keine unerwünschte irreversible Aggregierung der Polymerteilchen. Die Polymerpulver lassen sich daher leicht wieder in einer wässrigen Phase redispergieren.

Das Verdampfen der flüchtigen Bestandteile erfolgt beispielsweise durch Sprühtrocknung der erfindungsgemässen farbmittelhaltigen Polymerdispersion in einem Warmluftstrom oder durch Gefriertrocknung. Verfahren zur Sprühtrocknung und zur Gefriertrocknung wässriger Polymerdispersionen sind dem Fachmann dem Prinzip nach bekannt.

Bei einer Sprühtrocknung wird beispielsweise so vorgegangen, dass man die zu trocknenden Polymerisatdispersionen in einem üblichen Trockenturm in einem Warmluftstrom versprüht. Hierbei liegt die Eingangstemperatur des Warmluftstroms im Bereich von 100 bis 200 °C, vorzugsweise 120 bis 160 °C, und die Ausgangstemperatur des Warmluftstroms im Bereich von 30 bis 90 °C und vorzugsweise 60 bis 80 °C. Das Versprühen der wässrigen Polymerisatdispersion im Warmluftstrom kann beispielsweise mittels Ein- oder Mehrstoffdüsen oder über eine rotierende Scheibe erfolgen. Die Abscheidung der Polymerisatpulver erfolgt normalerweise unter Verwendung von Zyklonen oder Filterabscheidern. Die versprühte, wässrige Polymerisatdispersion und der Warmluftstrom werden vorzugsweise parallel geführt.

Selbstverständlich kann man den erfindungsgemässen Polymerdipsersionen vor dem Trocknungsprozess auch Trocknungshilfsmittel zusetzten, die auch als Sprühhilfsmittel bezeichnet werden. Hierzu kommen neutrale und anionische, wasserlösliche Polymere in Betracht. Diese haben in der Regel ein gewichtsmittleres Molekulargewicht M_{N} im Bereich von 1000 bis 100000, vorzugsweise 2000 bis 100000.

Konkrete Beispiele für neutrale Polymere sind: Polyvinylalkohole (siehe z. B. EP-A-56 622, EP-A-680 993, DE-A-22 14 410 und DE-A-26 14 261), Polyvinylpyrrolidone (siehe z. B. DE 22 38 903 und EP 576 844). Beispiele für anionische Polymere sind Phenolsulfonsäure-Formaldehyd-Kondensate (siehe z. B. EP-A 407 889, WO 98/03576), Naphthalinsulfonsäure-Formaldehyd-Kondensate (siehe z. B. WO 98/03577), Homo- und Copolymerisate der 2-Acrylamido-2-methylpropansulfonsäure (siehe z. B. EP-A 629 650, EP-A 671 435 und DE-A 195 39 460), Homo und Copolymere ethylenisch ungesättigter Carbonsäuren, wie insbesondere Acrylsäure, Methacrylsäure und Maleinsäure mit Hydroxyalkylestern (siehe z.B. JP 59 162 161).

Die vorliegende Erfindung betrifft ausserdem kosmetische Mittel, die wenigstens ein farbmittelhaltiges Polymerisat PF in Form einer wässrigen Polymerdispersion eines farbmittelhaltigen Polymerpulvers und die für kosmetische Mittel üblichen Adjuvantien enthalten.

Beispiele für kosmetische Mittel sind Mittel zur Behandlung der Gesichtshaut, insbesondere im Augenbereich, wie Kajal-Stifte, Eyeliner-Stifte, Augenbrauenstifte, Lidschatten, Cremerouge, Puderrouge, Make-up, Schminke, z. B. Theaterschminke, Lippenstifte; Mittel zur Behandlung von Augenbrauen und Wimpern, wie Mascara und Augenwimpernschminke; Nagellacke sowohl auf Lösungsmittelbasis als auch auf Wasserbasis; Haarbehandlungsmittel, wie Haargele, z. B. Wet-Gel, Stylinggel, Haarsprays, Haarmascara, Stylingmousse, Haarschaum, Haarshampoo; weiterhin farbige Seifen sowie Sonnenschutzmittel, z. B. Sun-Block-Cremes und Sun-Block-Stifte. Letztere sind eine bevorzugte Ausgestaltung der erfindungsgemässen kosmetischen Mittel und enthalten als Farbmittel F wenigstens einen der obengenannten UV-Absorber.

Bei kosmetischen Mitteln auf Wasserbasis, beispielsweise wässrigen Nagellacken, Mascara, Make-ups vom Typ O/W oder Make-ups vom Typ W/O, bei dekorativen Haarpflegemitteln, wie Wet-Gel, Stylinggel, Haarspray, Haarmascara, oder Stylingmousse wird man der Einfachheit halber vorzugsweise wässrige Dispersionen der farbmittelhaltigen Polymerisate PF einsetzen. Hingegen wird man bei kosmetischen Mitteln, die ausschließlich aus Ölen oder Fetten bestehen, insbesondere solche, die eine feste Form haben, z. B. Stifte, wie Kajal-Stifte, Eyeliner-Stifte, Augenbrauenstifte, stiftförmige Theaterschminke, Lippenstifte und ähnliche, sowie bei pulver- oder puderförmigen kosmetischen Mitteln wie Lidschatten und Cremerouge oder loses Puderrouge ein pulverförmiges, farbmittelhaltiges Polymerisat PF einsetzen.

Die Menge an farbmittelhaltigem Polymerisat PF in dem kosmetischen Mittel richtet sich in erster Linie nach dem gewünschten Farbeindruck, den das dekorative kosmetische Mittel aufweisen soll. Je nach Art des kosmetischen Mittels und des gewünschten Farbeindrucks liegt der Gehalt an farbmittelhaltigem Polymerisat in dem kosmetischen Mittel im Bereich von 0,1 bis 50 Gew.-%, bezogen auf das Gesamtgewicht des kosmetischen Mittels.

Bei Nagellacken wird man beispielsweise 1 bis 10 Gew.-%, bezogen auf den Nagellack, an farbmittelhaltigem Polymerisat PF einsetzen. Bei Mascara und Augenwimpernschminken setzt man in der Regel 2 bis 20 Gew.-%, bezogen auf das kosmetische Mittel, wenigstens eines farbmittelhaltigen Polymerisats PF als farbgebenden Bestandteil ein. In Kosmetikstiften, wie Kajal-Stiften, Eyeliner-Stiften, Augenbrauen-Stiften, Eye-Shadow-Stiften wird man in der Regel 10 bis 40 Gew.-% farbmittelhaltiges Polymerisat einsetzen. Bei Lidschatten liegt der Gehalt an Polymerisat PF in der Regel noch höher und kann bis zu 50 Gew.-% betragen. Bei Cremerouge und losem Puderrouge liegt der Gehalt an farbmittelhaltigem Polymerisat PF häufig im Bereich von 1 bis 20 Gew.-%, insbesondere im Bereich 2 bis 15 Gew.-%. Bei Lippenstiften wird man häufig je nach gewünschtem Farbeindruck 2 bis 40 Gew.-%, insbesondere 5 bis 30 Gew.-% farbmittelhaltiges Polymerisat, bezogen auf das Gesamtgewicht des Lippenstiftes einsetzen. Bei Haargel und Stylinggel sowie bei Haarspray wird man in der Regel geringere Gehalte an farbmittelhaltigem Polymerisat PF einsetzen, z. B. 0,1 bis 10 Gew.-%, insbesondere 0,5 bis 5 Gew.-%.

Selbstverständlich kann man zusätzlich auch andere Pigmente des Standes der Technik einsetzen, wobei diese teilweise die farbmittelhaltigen Polymerisate PF ersetzen oder diese zur Veränderung des Farbeindrucks ergänzen können. Die Menge an zusätzlichen Pigmenten des Standes der Technik liegt bei den erfindungsgemäßen kosmetischen Mitteln in der Regel im Bereich von 0,1 bis 30 Gew.-%, bezogen auf das Gesamtgewicht des kosmetischen Mittels und richtet sich naturgemäß nach der Art des kosmetischen Mittels und nach dem gewünschten Farbeindruck.

Die erfindungsgemäß zur Anwendung kommenden, feinteiligen, farbmittelhaltigen Polymerisate PF haben gegenüber den Pigmenten des Standes der Technik zum einen den Vorteil, dass sie sich leichter in die kosmetischen Mittel einarbeiten lassen, da ein Anreiben und Aufschließen des Pigments nicht erforderlich ist. Dies gilt sowohl für die wässrigen Polymerdispersionen als auch für die Pulver des Polymerisats PF. Der mögliche Einsatz wässriger Polymerdispersionen erleichtert insbesondere auch die Herstellung solcher Formulierungen, die einen hohen Wasseranteil und einen geringen oder gar keinen Fettanteil aufweisen. Zudem weisen die farbmittelhaltigen Polymerisate PF eine höhere Farbtiefe als vergleichbare Pigmente des Standes der Technik auf. Im Unterschied zu dem Pigmenten des Standes der Technik bedarf es bei einer Veränderung des Farbtons des kosmetischen Mittels nicht einer speziellen Anpassung der übrigen Bestandteile an den neuen farbgebenden Bestandteil. Da es möglich ist, in einem Polymermatrix-Typ die unterschiedlichsten Farbmittel einzubauen, kann man bei einmaliger Anpassung der Polymermatrix an das jeweilige kosmetische Mittel eine breite Palette von Farbmitteln zur Verfügung stellen.

Das erfindungsgemäße kosmetische Mittel kann als Suspension oder Dispersion in Lösungsmitteln oder Fettkörpern, als Emulsion, wie z. B. als Creme oder als Milch, als Pomade, Gel oder als fester Stift vorliegen; es kann als Aerosol konditioniert sein oder als Schaum vorliegen.

Das kosmetische Mittel enthält die für den jeweiligen Mittel-Typ üblichen kosmetischen Adjuvanzien, wie Verdickungsmittel, weichmachende Mittel, Hydratisierungsprodukte, grenzflächenaktive Mittel, Konservierungsmittel, Sequestriermittel, Antioxidationsmittel, Antischaummittel, Öle, Wachse, Lanolin, Parfums, Treibmittel, Farbmittel, Vitamine oder andere in der Kosmetik gewöhnlich verwendete Ingredienzien.

Beispiele für übliche kosmetische Ingredienzien sowie zahlreiche Formulierungsbeispiele für kosmetische Mittel sind in K. Schrader, Grundlagen und Rezepturen der Kosmetika, 2. Auflage, Hüthig-Buchverlag, Heidelberg, 1989, genannt.

Ist das Mittel als Aerosol konditioniert, verwendet man klassische Treibmittel, wie Alkane, Distickstoffoxid und Dimethylether.

Zu den Adjuvanzien, welche prinzipiell in den kosmetischen Mitteln der Erfindung vorhanden sind, zählen Lösungsmittel, wie Wasser, niedrige Monoalkohole oder Polyole mit 1 bis 6 Kohlenstoffatomen oder Mischungen davon; die besonders bevorzugten Monoalkohole oder Polyole sind Ethanol, i-Propanol, Propylenglycol, Glycerin und Sorbit; des Weiteren sind vorhanden Fettkörper, wie mineralische, tierische, pflanzliche oder synthetische Öle oder Wachse, Fettsäuren, Fettsäureester, wie Triglyceride von C₆-C₁₂-Fettsäuren, Fettalkohole, Vaseline, Paraffin, Lanolin, hydriertes Lanolin, acetyliertes Lanolin und Siliconöl.

Emulsionen in Form einer Creme oder eines Make-ups enthalten außer den Polymerisaten PF Fettalkohole, Fettsäureester und insbesondere Fettsäuretriglyceride, Fettsäuren, Lanolin und Derivate davon, natürliche oder synthetische Öle oder Wachse und Emulgatoren in Anwesenheit von Wasser.

Die Konzentration des Emulgatorsystems beträgt in der Regel 4 bis 35 %, bezogen auf das Gesamtgewicht der Emulsion; die Fettphase macht häufig zwischen 10 und 90 % aus und die wässrige Phase zwischen 10 und 90 %, bezogen auf das Gesamtgewicht der Emulsion. Bei den Emulgatoren handelt es sich um diejenigen, welche in diesem Emulsionstyp üblicherweise verwendet werden. Sie werden insbesondere ausgewählt unter:
- C₁₂-C₁₈-Sorbitan-Fettsäureestern,
- Estern von Hydroxystearinsäure und C₁₂-C₃₀-Fettalkoholen,
- Mono- und Diestern von C₁₂-C₁₈-Fettsäuren und Glycerin oder Polyglycerin,
- Kondensaten von Ethylenoxid und Propylenglycolen,
- oxypropylenierten/oxyethylenierten C₁₂-C₂₀-Fettalkoholen,
- polycyclischen Alkoholen, wie Sterolen,
- aliphatischen Alkoholen mit einem hohen Molekulargewicht, wie Lanolin,
- Mischungen von oxypropylenierten/polyglycerinierten Alkoholen und Magnesiumisostearat,
- Succinestern von polyoxyethylierten oder polyoxypropylenierten Fettalkoholen,
- Magnesium-, Calcium-, Lithium-, Zink- oder Aluminiumlanolat und -stearat, gegebenenfalls als Mischung mit hydriertem Lanolin, Lanolinalkohol, oder Stearinsäure oder Stearylalkohol.

Zu den Fettprodukten, welche die Fettphase der Emulsionen bilden, zählen:
- Kohlenwasserstoff-Öle, wie Paraffinöl, Purcellinöl, Perhydrosqualen und Lösungen mikrokristalliner Wachse in diesen Ölen,
- tierische oder pflanzliche Öle, wie Süßmandelöl, Avocadoöl, Calophylumöl, Lanolin und Derivate davon, Ricinusöl, Pferdeöl, Schweineöl, Sesamöl, Olivenöl, Jojobaöl, Karité-Öl, Hoplostethus-Öl,
- mineralische Öle, deren Destillationsbeginn unter Atmosphärendruck bei ca. 250 °C und deren Destillationsendpunkt bei 410 °C liegt, wie z. B. Vaselinöl,
- Ester gesättigter oder ungesättigter Fettsäuren, wie Alkylmyristate, z. B. i-Propyl-, Butyl- oder Cetylmyristat, Hexadecylstearat, Ethyl- oder i-Propylpalmitat, Octan- oder Decansäuretriglyceride und Cetylricinoleat.

Die Fettphase kann auch in anderen Ölen lösliche Siliconöle, wie Dimethylpolysiloxan, Methylphenylpolysiloxan und das Siliconglycol-Copolymer, Fettsäuren und Fettalkohole enthalten.

Um die Retention von Ölen zu begünstigen, kann man auch Wachse verwenden, wie z. B. Carnauba-Wachs, Candellilawachs, Bienenwachs, mikrokristallines Wachs, Ozokeritwachs und Ca-, Mg- und Al-Oleate, -Myristate, -Linoleate und -Stearate.

Die Emulsionen können auch in Form eines Stiftes vorliegen. In diesem Fall beträgt die Konzentration der Wasserphase in der Emulsion im Allgemeinen 5 bis 70 Gew.-%, bezogen auf das Gesamtgewicht der Emulsion.

Fettgele enthalten in der Regel ein Öl oder ein Wachs und ein Verdickungsmittel, wie Kieselerde. Die ölig-alkoholischen oder wässrig-alkoholischen Gele enthalten einen oder mehrere niedrige Alkohole und Polyole, wie Ethanol, Propylenglycol oder Glycerin, ein Verdickungsmittel, wie Kieselerde, Cellulosederivate, Polyacrylsäurederivate und Guar-, Carouba- und Xanthangummi in Anwesenheit von Öl bzw. von Wasser.

Feste Stifte bestehen in der Regel aus Fettkörpern, wie natürlichen oder synthetischen Wachsen und Ölen, Fettalkoholen, Fettsäureestern und Lanolin.

Kosmetische Mittel auf Wasserbasis enthalten häufig Gelbildner, wie Hydrokolloide und halbfeste Fette und Wachse, z. B. Guargummi, Xanthangummi, Tragant, Alginate, Stärke, Stärkederivate, Gelatine, Cellulose und Cellulosederivate, wie Methylcellulose, Natrium-Carboxymethylcellulose, Hydroxypropylcellulose, Hydroxyethylcellulose und Polyacrylate.

Die im Folgenden angegebenen Beispiele für farbmittelhaltige _{P}olymerdispersionen und farbmittelhaltige Pulver sowie die kosmetischen Formulierungsbeispiele sollen die vorliegende Erfindung näher erläutern:

### I. Allgemeines

### 1. Analytik

Die Bestimmung der Polymerisatteilchengröße (d_{z}-Wert) erfolgte in der oben beschriebenen Weise mittels eines Coulter N4 Plus Particle Analyzers an 0,01 gew.-%igen Proben der Dispersion.

Die relative Lichtdurchlässigkeit (LD-Wert) für weisses Licht wurde an einer 0,01 gew.-%igen Probe bei einer Schichtdicke von 2,5 cm gegen Wasser bestimmt.

Die Viskosität wurde in einem Eprecht-Viskosimeter A-III bei 23 °C für 20 min bestimmt.

### 2. Eingesetzte Farbmittel:

- UV-Absorber 1:: 4-Methoxyzimtsäure-n-octylester
- UV-Absorber 2:: 4,4-Diphenylbutadien-1,1-dicarbonsäurebis(2-ethylhexyl)ester,
- UV-Absorber 3:: 4-tert.-Butyl-4'-methoxydibenzoylmethan,
- UV-Absorber 4:: 2,4,6-Tris-{N-[4-(2-ethylhex-1-yl)oxycarbonylphenyl]amino}-1,3,5-triazin,
- UV-Absorber 5:: 2-Cyano-3,3-diphenylacrylsäure-(2'-ethylhexyl)ester (Octocrylen)

### 3. Nichtionisches oberflächenaktives Mittel:

- NO1: Polyethoxiliertes, hydriertes Castor-Öl (EMANON CH25 der KAO Corporation Japan).

### 4. Eingesetzte amphiphile Polymere PA:

- Polymer PA 1:: Natriumsalz eines Copolymers aus Maleinsäure und Diisobuten im Molverhältnis 1:1, worin ein Teil der Carboxylgruppen mit ethoxilierten C_{13/15}-Oxoalkohol (Ethoxilierungsgrad 7) verestert sind als wässrige Lösung (45 Gew.-%). Das Polymer hat einen K-Wert von 20 (bestimmt nach Fikentscher als 1 gew.-%ige Lösung in N,N-Dimethylformamid), entsprechend einem Molekulargewicht von etwa 3000 und eine Säurezahl von 440 mg KOH/g Polymer.
- Polymer PA 2:: Salz eines Copolymers aus tert.-Butylacrylat, Ethylacrylat und Methacrylsäure im Molverhältnis 59:11:30 mit 2-Amino-2-methylpropanol als wässrige Lösung (14 Gew.-%). Das Polymer hat einen K-Wert von 37 (bestimmt nach Fikentscher als 1 gew.-%ige Lösung in Ethanol) und eine Säurezahl von 140 bis 160 mg KOH/g Polymer.
- Polymer PA 3:: Salz eines Copolymers aus Vinylacetat und Crotonsäure im Molverhältnis 9:1 mit 2-Amino-2-methylpropanol als wässrige Lösung (18 Gew.-%). Das Polymer hat einen K-Wert von 34 (bestimmt nach Fikentscher als 1 gew.-%ige Lösung in Ethanol) und eine Säurezahl von 67,6 mg KOH/g Polymer.
- Polymer PA 4:: Salz eines Copolymers aus tert.-Butylacrylamid, Ethylacrylat und Acrylsäure im Molverhältnis 43,5:44,5:12 mit 2-Amino-2-methylpropanol als wässrige Lösung (12 Gew.-%). Das Polymer hat einen K-Wert von 40 (bestimmt nach Fikentscher als 1 gew.-%ige Lösung in Ethanol) und eine Säurezahl von 140 bis 160 mg KOH/g Polymer.

### II. Herstellung wässriger Dispersionen UV-Absorber enthaltender Polymerisate (Beispiele 1 bis 5):

### 1. Herstellung der Miniemulsion (allgemeine Vorschrift)

In einem Reaktionsgefäß mit Rührer legte man entionisiertes Wasser, 1,2 g nichtionische oberflächenaktive Mittel NO1 und das jeweilige Polymer PA (als wässrige Lösung) vor. Die Gesamtmenge an Wasser betrug etwa 500 ml. Hierzu gab man innerhalb 2 Minuten eine Lösung des jeweiligen UV-Absorbers in den zu polymerisierenden Monomeren (Monomer/Farbmittellösung). Die Einsatzmengen und Art des amphiphilen Polymers PA sowie die Bestandteile dieser Lösung sind in Tabelle 1 angegeben. Die Zusammensetzung der Monomerlösung war in allen Beispielen wie folgt: 5 g Stearylacrylat, 5 g Butandioldiacrylat, 95 g Methylmethacrylat und 20 g UV-Absorber. Anschließend rührte man weitere 10 Minuten. Die dabei resultierenden, konventionellen, farbstoffhaltigen Monomeremulsionen wurden anschließend wie folgt zu einer wässrigen Monomermikroemulsion mittels Ultraschall homogenisiert.

Als Ultraschallquelle diente ein Branson Sonifier II 450. Die Ultraschallbehandlung erfolgte unter Rühren der Emulsion durch 5-minütige Beschallung bei der Einstellung duty-cycle 25%, output 10, und 10 minütige Beschallung bei der Einstellung duty cycle 100% und output 10. Die Tröpfchengrösse (Volumenmittel) in der Monomeremulsion lag unterhalb 400 nm.

### 2. Polymerisation der farbstoffhaltigen Miniemulsion (allgemeine Vorschrift)

Die nach 1. erhaltene Miniemulsion wurde in einem Polymerisationsgefäß vorgelegt und auf 80 °C erwärmt. Dann gab man unter Rühren in einer Portion die Initiatorlösung (1 g Natriumperoxodisulfat in 38 ml Wasser) zu, ließ 3,5 h bei 80 bis 85 °C nachreagieren, kühlte dann auf 25 °C ab und filtrierte zur Bestimmung des Koagulatanteils über ein 75 µm Sieb. Man erhielt eine etwa 20 gew.-%ige wässrige Dispersion des Polymerisats PF. Der Anteil der Koagulatbildung war in allen Fällen gering (< 5 %, bezogen auf die Einsatzstoffe).

Die Lichtdurchlässigkeit (LD-Wert), der pH-Wert, die Viskosität und die Lagerstabilität der so erhaltenen Polymerdispersionen sind in Tabelle 1 angegeben. Die mittlere Teilchengröße der Polymerisatteilchen lag herstellungsbedingt unterhalb 400 nm.

**Tabelle 1:**

| UV-Absorber enthaltende Polymerdispersionen | | | | | | | | |
|---|---|---|---|---|---|---|---|---|
| Bsp. | Polymer PA Typ [g] | | UV-Absorber | FG ¹⁾[Gew.-%] | LD ²⁾ [%] | pH | Viskosität [mPas] | Lagerstabilität ³⁾ |
| 1 | 1 | 18 | UV1 | 20,2 | 98,3 | 6,8 | 3,7 | 3 |
| 2 | 1 | 9 | UV1 | 19,4 | 96,4 | 6,5 | 3,7 | 2 |
| 3 | 1 | 18 | UV3 | 20,5 | 97,5 | 6,7 | 3,7 | 2 |
| 4 | 1 | 18 | UV4 | 21,5 | 96,8 | 7,0 | 3,7 | 2 |
| 5 | 2 | 18 | UV1 | 20,7 | 59,2 | 7,8 | n.b. | 2 |

| | | | | | | | | |
|---|---|---|---|---|---|---|---|---|
| 1) FG=Feststoffgehalt | | | | | | | | |
| 2) LD-Wert (Lichtdurchlässigkeit) | | | | | | | | |
| 3) Lagerstabilität wurde nach einem Monat Lagerung bei Raumtemperatur anhand der Koagulatbildung nach der folgenden Skala beurteilt: 1: viel Koagulat, geringe Lagerstabilität 2: geringe Koagulatmenge, lagerstabil 3: kein Koagulat, sehr gute Lagerstabiliät | | | | | | | | |

In gleicher Weise führte auch die Verwendung von 18 g Polymer PA3 und von 18 g Polymer PA4 anstelle von PA1 zu lagerstabilen Polymerdispersionen.

### Redispergierbarkeit:

Alle Polymerisate PF ließen sich unter Kühlung im Trockeneis/Acetonbad durch Anlegen von Vakuum zu Pulvern gefriertrocknen. Diese Pulver liessen sich in Mineralöl, 1,3-Butylenglykol und Propylenglykol unter heftigem Rühren redispergieren.

Im Folgenden sind beispielhaft Formulierungen von Polymerisaten PF in kosmetischen Pflegemitteln beschrieben. Alle Angaben sind in Gramm. Die Mengenangaben der wässrigen Dispersionen des farbstoffhaltigen Polymerisats PF sind auf den Polymerisatanteil bezogen.

### Nagellackformulierungen (Formulierung 1 bis 3)

| Formulierung 1: | |
|---|---|
| 26,3 | Nitrocellulose |
| 4,9 | Copolymer aus Polyoxyisobutylen und Methylenharnstoff |
| 7,8 | Copolymer aus Butylacrylat und Vinylisobutylether, |
| | 50 Gew.-% in Essigester (Acronal®700 L 50 % BASF) |
| 4,9 | Methoxypropylacetat |
| 53,5 | Butylacetat |
| 2,6 | Polymerisat PF als wässrige Dispersion |

| Formulierung 2: | |
|---|---|
| 16,0 | Nitrocellulose |
| 4,0 | Toluolsulfonamid-Formaldehyd-Harz |
| 5,0 | Dibutylphthalat |
| 10,0 | Butylacetat |
| 10,0 | Ethylacetat |
| 10,0 | Ethanol |
| 40,0 | Toluol |
| 5,0 | Polymerisat PF als wässrige Dispersion |

Die Bestandteile mit Ausnahme der wässrigen Dispersion von PF werden gelöst. Dann wird das farbstoffhaltige Polymerisat PF als wässrige Dispersion eingerührt und die Mischung anschließend homogenisiert.

### Wässriger Nagellack: (Formulierungen 3 bis 4)

| Formulierung 3: | |
|---|---|
| 27,2 | Wässrige Polyurethan-Dispersion |
| 13,8 | Acrylsäure-Styrol-Copolymer |
| 0,08 | Polyacrylsäure-Verdicker |
| 0,5 | Butylglykolacetat |
| 2,4 | Polymerisat PF als wässrige Dispersion |
| | Wasser ad 100 |

Das Polyurethan wird als feindisperse wässrige Dispersion vorgelegt. Das Acrylsäure-Styrol-Copolymer wird als wässrige Dispersion unter Rühren hinzugefügt. Anschließend gibt man unter Rühren den Acrylatverdicker zu. Es wird weiter gerührt, bis die Masse hochviskos ist. Schließlich rührt man die wässrige Dispersion von PF ein.

### Formulierung 4:

Wie Formulierung 3, jedoch mit 0,4 g Acid Blue 74 Aluminium Lake und 2,0 g Polymerisat PF als wässrige Dispersion.

### Mascara

| Formulierung 5: | |
|---|---|
| 14,0 | demin. Wasser |
| 0,2 | Antioxidans (Oxynex 2004 der E. Merck, Darmstadt) |
| 2,5 | Polyoxyethylen/Polyoxypropylen-Blockcopolymer (Poloxamer 407 der BASF Aktiengesellschaft) |
| 3,5 | Polyvinylpyrrolidon |
| 11,0 | Ethanol |
| 0,7 | Triethanolamin |
| 0,52 | Polyacrylsäure (CTFA: Carbomer) |
| 57,58 | demin. Wasser |
| 10,0 | Polymerisat PF als wässrige Dispersion |

Man lässt die Polyacrylsäure in Wasser quellen und arbeitet dann unter Rühren die klar gelösten restlichen Bestandteile zu einem Gel ein. Anschließend arbeitet man die wässrige Dispersion von PF ein.

### Augenwimpernschminke

| Formulierung 6: | |
|---|---|
| 80,8 | Kastoröl |
| 6,0 | Capryl/Caprin-Triglycerid |
| 0,2 | Antioxidans (Oxynex 2004 der E. Merck, Darmstadt) |
| 2,0 | Trihydroxystearin |
| 0,3 | Polyvinylpyrrolidon |
| 2,0 | Sorbitanoleat |
| 8,7 | Polymerisat PF als Pulver |

Die Fettbestandteile werden ineinander gelöst. Anschließend rührt man Polyvinylpyrrolidon ein. Dann wird das pulverförmige Polymerisat PF untergemischt.

### Creme Mascara

| Formulierung 7: | |
|---|---|
| 75,0 | Petroleum-Destillat |
| 8,3 | Quaternium-18-Hectorit |
| 2,5 | Propylencarbonat |
| 11,5 | wässrige Dispersion von PF |
| 1,0 | Ultramarin |
| 1,7 | Vinylpyrrolidon/Vinylacetat-Copolymer |

Die Komponenten der Fettphase werden mittels starker Scherkräfte zu einem Gel verarbeitet. Anschließend arbeitet man die wässrige Dispersion des farbstoffhaltigen Polymerisats PF und das Vinylpyrrolidon/Vinylacetat-Copolymer ein und homogenisiert.

### Kajalstift-Kosmetikstift

| Formulierung 8: | |
|---|---|
| 34,3 | Hydroxyliertes Lanolin |
| 17,10 | Hydriertes Coco-Glycerid |
| 2,9 | Lanolin |
| 28,6 | Glycerylstearate |
| 17,1 | Polymerisat PF als Pulver |

Die Fettkomponenten werden bei 80 °C aufgeschmolzen. Anschließend wird das pulverförmige Polymerisat PF untergemischt, gegebenenfalls parfümiert, durch Gießen oder Extrudieren zu Minen für Kosmetikstifte geformt.

### Eye-Liner-Stift

| Formulierung 9: | |
|---|---|
| 30,0 | Cyclomethicone |
| 6,7 | Lanolinöl |
| 8,0 | Carnauba Wax |
| 3,3 | Bienenwachs |
| 22,7 | Paraffinöl |
| 2,7 | Cetylalkohol |
| 20,0 | Polymerisat PF als Pulver |
| 5,6 | Pigment Blue 15 |
| 1,0 | Eisenoxidpigment |

### Augenbrauenstift

| Formulierung 10: | |
|---|---|
| 78,0 | Cutina LM (Lippenstiftmasse der Firma Henkel KGaA, Düsseldorf) |
| 12,0 | Ozokerite |
| 9,0 | Polymerisat PF als Pulver |
| 1,0 | Eisenoxidpigment |

### Lidschatten

| Formulierung 11 | |
|---|---|
| 20 | Talkum |
| 10 | Kartoffelstärke |
| 5 | Magnesiumstearat |
| 45 | Polymerisat PF als Pulver |
| 5 | Ultramarin (Sicomet Blau P 77007) |
| 15 | Lidschatten Binder |

### Lidschatten Binder

| | |
|---|---|
| 35 | Lanolin |
| 30 | Isopropylstearat |
| 30 | Paraffinöl |
| 3 | Parfümöl |
| 1 | Carnauba Wax |
| 1 | Propylparaben |

Die Lidschattenbestandteile werden homogen gemischt, und das pulverförmige Polymerisat PF und das Farbpigment (Ultramarin) eingerührt. Die Binderbestandteile werden bei 70 °C geschmolzen. Die Lidschattenbestandteile werden mit dem geschmolzenen und gut vermengten Binder besprüht. Danach wird bei einem Pressdruck von 40 bis 60 bar gepresst. Man erhält einen Lidschattenpuder mit weichem Hautfeeling und einzigartigem Farbeffekt.

### Formulierung 12:

Wie vorherige Formulierung, aber mit 50 g Pulver von PF statt der Ultramarin/PF-Mischung.

### Lidschatten in Stiftform

| Formulierung 13: | |
|---|---|
| 15,0 | Triglycerid einer C₁₈₋₃₆-Säure |
| 5,0 | Glycerylbehenat |
| 35,0 | Mineralisches Öl |
| 15,0 | Mineralisches Öl (und) Lanolin Alkohol |
| 0,2 | Parfümöl |
| 0,8 | Polyvinylpyrrolidon |
| 1,5 | Talkum |
| 27,5 | Polymer PF als Pulver |

Die Fettkomponenten werden bei 80 °C geschmolzen und das pulverförmige Polymerisat PF untergemischt. Anschließend wird parfümiert und die Masse durch Gießen oder Extrudieren zu Minen für Kosmetikstifte geformt.

### Eye-Shadow-Stift

| Formulierung 14: | |
|---|---|
| 6,0 | Bienenwachs |
| 5,0 | Carnauba Wax |
| 10,0 | Candelilla Wax |
| 34,0 | Hexyllaurat |
| 20,0 | Kastoröl |
| 20,0 | Polymer PF als Pulver |
| 4,0 | Chromoxid-Grün-Pigment |
| 1,0 | Parfümöl |

Lidschattenstifte aus den beiden obigen Formulierungen können auch anstelle von reinem pulverförmigem Polymerisat PF mit Mischungen von Farbpigmenten und pulverförmigen Polymerisaten PF formuliert werden.

### Cremerouge (Formulierungen 15 und 16)

| Formulierung 15: | |
|---|---|
| 5,5 | Candelilla Wax |
| 8,5 | Bienenwachs |
| 3,0 | Cetylpalmitat |
| 8,5 | Paraffinöl |
| 43,0 | Cetearyloctanoat |
| 3,0 | Hydrierte Kokosfettsäureglyceride |
| 11,0 | Vaseline |
| 14,5 | Talkum |
| 3,0 | Polymerisat PF als Pulver |

Die Bestandteile der Grundmasse werden auf etwa 80 °C erhitzt und gut vermischt. Anschließend wird das pulverförmige Polymerisat PF in die Grundmischung eingearbeitet.

### Formulierung 16:

Wie Formulierung 17, jedoch statt des reinen Pulvers PF werden 0,5 g Pigment Red 57:1 und 2,5 g Pulver PF eingearbeitet.

### Loses Puderrouge (Formulierungen 17 bis 19)

| Formulierung 17: | |
|---|---|
| 77,0 | Talkum |
| 10,0 | Magnesiumstearat |
| 2,0 | Calciumcarbonat |
| 0,5 | Vaseline |
| 0,5 | Paraffinöl |
| 10,0 | Polymerisat PF als Pulver |

Die trockenen Puderbestandteile werden homogen gemischt und mit den geschmolzenen und gut vermengten Fettbestandteilen gemischt.

### Formulierung 18:

Wie Formulierung 17, jedoch kann für intensivere Rotfärbung das reine pulverförmige Polymerisat PF durch eine Mischung von 1 bis 2 g Rotpigment, z. B. Pigment Red 172 Aluminium Lake und 8 bis 9 g pulverförmiges PF ersetzt werden.

### Formulierung 19:

Wie Formulierung 18, jedoch mit 9,5 g pulverförmigem Polymerisat PF und 0,5 g Eisenoxid-Pigment.

### Make up Typ W/O

| Formulierung 20: | |
|---|---|
| 5,5 | Hydriertes Kastoröl, ethoxyliert mit 7 EO-Einheiten |
| 7,0 | Cetearyloctanoat |
| 4,5 | Isopropylmyristat |
| 14,0 | Paraffinöl |
| 0,3 | Magnesiumstearat |
| 0,3 | Aluminiumstearat |
| 2,0 | PEG-45/Dodecylglykol-Copolymer |
| 0,2 | Propylparaben |
| 5,0 | Propyleneglykol |
| 0,6 | Magnesium Sulfate |
| 0,1 | Paraben |
| 50,8 | Wasser |
| 0,2 | Parfümöl |
| 0,5 | Vitamin E-Acetat |
| 9,0 | wässrige Dispersion von PF (gerechnet als Feststoff) |

Die Bestandteile der Fettphase und der Wasserphase erhitzt man separat auf etwa 75 °C und bringt dann die Wasserphase unter Rühren langsam in die Fettphase ein. Nach Homogenisieren kühlt man unter Rühren auf 40 °C ab, gibt Parfümöl und Wirkstoffe zu, und homogenisiert nochmals. Dann wird die wässrige Dispersion von PF untergerührt.

### Formulierung 21:

Wie Formulierung 20, jedoch mit 8 g Polymerisat PF, 0,5 g Eisenoxid-Pigment und 0,5 g Titandioxid-Pigment.

### Make up Typ O/W

| Formulierung 22: | |
|---|---|
| 1,7 | Glycerylstearat |
| 1,7 | Cetylalkohol |
| 1,7 | Ceteareth-6, Stearylalkohol |
| 1,7 | Ceteareth-25 |
| 5,2 | Capryl/Caprin-Triglycerid |
| 0,2 | Methyldibromoglutaronitril (und/oder) Phenoxyethanol |
| 0,3 | Imidazolidinyl-Harnstoff |
| 4,3 | Propyleneglykol |
| 69,0 | Demineralisiertes Wasser |
| 0,2 | Parfümöl |
| 14,0 | Polymerisat PF als wässrige Dispersion |

Die Bestandteile der Fettphase und der Wasserphase werden separat auf etwa 75 °C erhitzt. Dann bringt man die Wasserphase zusammen mit der wässrigen Dispersion von PF unter Rühren langsam in die Fettphase ein. Man homogenisiert und kühlt unter Rühren auf 40 °C ab und gibt nach Belieben Parfümöl zu und homogenisiert nochmals.

### Formulierung 23:

Wie vorheriges Formulierung, jedoch mit 12 % wässrige Dispersion von PF, 1,5 % Iron oxides und 0,5 % Titanium dioxide.

### Theaterschminke

| Formulierung 24: | |
|---|---|
| 75,0 | Petroleum Destillat |
| 8,3 | Quaternium-18-Hectorit |
| 2,5 | Propylencarbonat |
| 1,7 | Polyvinylpyrrolidon/Vinylacetat-Copolymer |
| 12,5 | Polymerisat PF als Pulver |

Aus den Bestandteilen wird unter Aufbietung starker Scherkräfte ein Gel hergestellt. Das Copolymer und pulverförmige Polymerisat PF wird eingearbeitet. Anschließend wird homogenisiert.

### Formulierung 25:

Wie vorheriges Formulierung 24, jedoch mit 11 g Polymerisat PF und 1,5 g konventionellem Farbpigment, z. B. Pigment Blue 15.

| Formulierung 26: | |
|---|---|
| 67,5 | Mineralisches Öl |
| 20,0 | Bienenwachs |
| 10,0 | Ceresin Wachs |
| 2,5 | Polymerisat PF als Pulver |

Fettkomponenten werden geschmolzen und mit pulverförmigen Polymerisat PF zu einer homogenen Paste verarbeitet.

### Fettschminke für das Theater in Stiftform

| Formulierung 27: | |
|---|---|
| 22,0 | Ceresin Wachs |
| 18,0 | Bienenwachs |
| 44,0 | Mineralisches Öl |
| 5,0 | Terpentin |
| 1,0 | Parfümöl |
| 8,0 | Polymerisat PF als Pulver |
| 2,0 | Eisenhexacyanoferrat |

Die Fettkomponenten werden bei 80 °C geschmolzen und das pulverförmige Polymerisat PF untergemischt. Anschließend wird die Masse parfümiert und durch Gießen oder Extrudieren zu Minen für _{K}osmetikstifte geformt.

### Lippenstift (Formulierungen 28 bis 31)

| Formulierung 28: | |
|---|---|
| 3,0 | Carnauba Wax |
| 3,5 | Candelilla Wax |
| 2,0 | Bienenwachs |
| 7,0 | Mikrokristallines Wachs |
| 1,5 | Cetylpalmitat |
| 5,0 | Vaseline |
| 3,5 | Lanolin-Wachs |
| 2,0 | Lanolin |
| 9,0 | Cetearyloctanoat |
| 0,2 | Bisabolol |
| 0,5 | Tocopherol |
| 2,0 | Tocopheryl-Acetat |
| 3,5 | Hydrierte Kokosfettsäureglyceride |
| 42,3 | Kastoröl |
| 15,0 | Polymerisat PF als Pulver |

Die Bestandteile der Fettmasse werden geschmolzen. Dann arbeitet man das pulverförmige Polymerisat PF in die Grundmasse ein und gießt die homogene Schmelze in auf 60 °C vorgewärmte Gießformen.

Die Gießlinge werden den Formen kalt entnommen und nach Erwärmen auf Raumtemperatur noch kurz abgeflammt.

| Formulierung 29: | |
|---|---|
| 14,0 | Oleylalkohol |
| 10,0 | Kastoröl |
| 6,0 | Diisopropyladipat |
| 5,0 | Stearamide MEA |
| 10,0 | Polymerisat PF als Pulver |
| 1,0 | Eisenoxid-Pigment |
| 9,0 | Stearylheptanoat |
| 7,0 | Isopropyllanolat |
| 8,0 | Carnauba Wax |
| 10,0 | Bienenwachs |
| 5,0 | Cetylalkohol |
| 5,0 | Ozokerite |
| 3,0 | Microkristallines Wachs |
| 2,0 | Polyethylen |
| 2,0 | Petrolatum |
| 2,0 | Mineralisches Öl |
| 1,0 | Parfümöl |

| Formulierung 30: | |
|---|---|
| 10,0 | Hydroxyoctacosanylhydroxystearat |
| 9,0 | Candelilla Wax |
| 25,0 | Kastoröl |
| 7,9 | Isopropylmyristat |
| 5,0 | Sorbitantrioleat |
| 3,0 | Hydroxyliertes Lanolin |
| 6,0 | Butyleneglykol |
| 0,1 | Propylparaben |
| 1,0 | Parfümöl |
| 3,0 | Ultramarin |
| 30,0 | Polymerisat PF als Pulver |

| Formulierung 31: | |
|---|---|
| 40,0 | Kastoröl |
| 10,0 | Mineralisches Öl |
| 9,0 | Hydriertes Kastoröl |
| 5,0 | Kakaobutter |
| 10,0 | Carnauba-Wax |
| 5,0 | Stearylheptanoat |
| 5,0 | Bienenwachs |
| 10,0 | Lanolin |
| 5,0 | Polymerisat PF als Pulver |
| 1,0 | Parfümöl |

### Haargel-Formulierungen (Formulierungen 32 bis 34)

| Formulierung 32: | |
|---|---|
| 59,8 | Wasser |
| 0,5 | Polyacrylsäure (CTFA: Carbomer) |
| 1,2 | Triethanolamin |
| 29,9 | Glycerin |
| 2,0 | Propylenglycol |
| 2,3 | Dimethicone-Copolyol |
| 0,3 | Imidazolidinyl-Harnstoff |
| 4,0 | Polymerisat PF als wässrige Dispersion |

| Formulierung 33: | |
|---|---|
| 0,7 | Polyacrylsäure (CTFA: Carbomer) |
| 92,1 | Wasser |
| 0,7 | Hydriertes Kastoröl, ethoxyliert mit 40 EO Einheiten |
| 0,2 | Parfümöl |
| 0,3 | Imidazolidinylharnstoff |
| 1,0 | Panthenol |
| 3,0 | Polyvinylpyrrolidon |
| 1,0 | Triethanolamin |
| 1,0 | Polymerisat PF als wässrige Dispersion |

| Formulierung 34 (Styling-Gel): | |
|---|---|
| 0,5 | Polyacrylsäure (CTFA: Carbomer) |
| 74,7 | Wasser |
| 15,0 | Ethanol |
| 0,2 | Hydroxyethylcetyldimonium-Phosphat |
| 6,0 | Polyvinylpyrrolidon |
| 0,3 | Imidazolidinylharnstoff |
| 0,8 | Tetrahydroxypropylethylendiamin |
| 2,5 | Polymerisat PF als wässrige Dispersion |

### Haarsprays (Formulierungen 35 bis 37)

| Formulierung 35: | |
|---|---|
| 3,0 | Polyvinylpyrrolidon |
| 4,0 | Vinylpyrrolidon/Vinylacetat-Copolymer |
| 0,7 | Rosin Acrylat |
| 44,3 | Ethanol |
| 3,0 | Polymerisat PF als wässrige Dispersion |
| 45,0 | Propan/Butan |

Die Komponenten werden mit Ausnahme der wässrigen Dispersion des Polymeren PF gelöst. Anschließend wird die wässrige Dispersion von PF eingerührt. Vor dem Abfüllen einige Glaskugeln zugeben.

| Formulierung 36: | |
|---|---|
| 1,5 | Acrylsäure/Acrylamid-Copolymer |
| 0,11 | Aminomethylpropanol |
| 0,02 | Cyclomethicone |
| 6,0 | Wasser |
| 3,0 | Polymerisat PF als wässrige Dispersion |
| 60,0 | Dimethylether |
| 29,37 | Ethanol |

### Formulierung 37:

Die Formulierung entspricht Formulierung 36, jedoch werden 2 g wässrige Dispersion von PF und 1 g Pigment Blue 15 eingearbeitet.

### Haarmascara (Formulierungen 38 bis 40)

| Formulierung 38: | |
|---|---|
| 15,0 | Mischung aus Bienenwachs, Carnauba (Copernicia Cerifera) Wax, Stearinsäure, Ceteareth-25, PEG-2 Stearate SE, mineralischem Öl, hydriertem Kokosöl und Cetylalcohol (Base RW 135, Wacker) |
| 1,5 | Dimethicone |
| 0,5 | Konservierungsmittel |
| 42,1 | Wasser |
| 0,45 | Triethanolamin |
| 0,45 | Xanthan, Hectorit und Cellulosegummi |
| 30,0 | Acrylsäure-Copolymer |
| 10.0 | Polymerisat PF als wässrige Dispersion |

### Formulierung 39:

Wie Formulierung 38, aber mit 8 g Polymerisat PF und 2 g Pigment Blue 15.

| Formulierung 40: | |
|---|---|
| 14,0 | demin. Wasser |
| 0,3 | Imidazolidinylharnstoff |
| 2,5 | Poloxamer 407 |
| 3,5 | Polyvinylpyrrolidon |
| 11,0 | Ethanol |
| 0,7 | Triethanolamin |
| 0,52 | Carbomer |
| 57,48 | demineralisiertes Wasser |
| 1,0 | Eisenoxidpigment |
| 9,0 | Polymerisat PF als wässrige Dispersion |

Die Komponenten werden als Gel formuliert, wobei man das Farbpigment und die wässrige Dispersion von PF zuletzt einrührt.

### Sunblock Stift

| Formulierung 41: | |
|---|---|
| 4,0 | Carnauba Wax |
| 4,0 | Candelilla Wax |
| 4,0 | Bienenwachs |
| 9,0 | Mikrokristallines Wachs |
| 1,0 | Cetylpalmitat |
| 10,0 | Lanolin Wax |
| 5,0 | Ethoxyliertes Lanolin-Öl, 75 Ethylenoxideinheiten |
| 5,0 | Cetearyloctanoat |
| 5,0 | Octylmethoxycinnamat |
| 38,1 | Capryl/Caprin-Triglycerid |
| 0,2 | Parfümöl |
| 2,0 | Titandioxid |
| 0,5 | Tocopherol |
| 2,0 | Tocopherylacetat |
| 0,2 | Bisabolol |
| 5,0 | Polymerisat PF mit UV-Absorber als Pulver |

Die Bestandteile der Fettmasse werden aufgeschmolzen. Dann rührt man Titandioxid ein. Bei 65 °C werden die Wirkstoffe und das pulverförmige Polymerisat PF in die Grundmasse eingearbeitet. Die homogene Schmelze wird in auf 60 °C vorgewärmte Gießformen gegossen.

### Farbige Seife:

| Formulierung 42: | |
|---|---|
| 92,9 | Seifenspäne |
| 2,0 | Polyquaternium-16 |
| 0,1 | Bisabolol |
| 0,4 | Tetrasodium EDTA |
| 2,0 | Parfümöl |
| 1,0 | PEG-6 |
| 1,6 | Water |

In 100 g der Seifengrundmasse aus genannten Bestandteilen 0,5 g wässrige Dispersion von PF einarbeiten.

| Formulierung 43: | |
|---|---|
| 4,2 | Natriumhydroxid |
| 5,6 | Wasser |
| 22,6 | Propylene Glycol |
| 5,2 | Cocoamide DEA |
| 10,4 | Cocaminoxid |
| 4,2 | Natriumlaurylsulfat |
| 7,3 | Myristinsäure |
| 16,6 | Stearinsäure |
| 5,2 | Tocopherylacetat |
| 18,7 | Glycerin |

Die Zutaten werden vermischt und bei 85 °C zu einer klaren Schmelze aufgeschmolzen. 100 Teile der Seifengrundmasse werden mit 3 Teilen wässriger Dispersion des Polymeren PF vermischt und die so erhaltene Masse noch heiß in Formen gegossen.

### Farbgebender Haarschaum (Formulierungen 44 und 45)

| Formulierung 44: | |
|---|---|
| 2,0 | Cocotrimoniummethosulfat |
| 0,2 | Parfümöl |
| 7,0 | Polyquaternium-64. |
| 2,0 | Polyquaternium-11 |
| 0,2 | Ceteareth 25 |
| 0,5 | Panthenol |
| 0,05 | Benzophenon-4 |
| 0,2 | Mischung aus Amodimethicon, Tallowtrimoniumchlorid und Nonoxynol 10 |
| 0,2 | Hydroxyethylcellulose |
| 15,0 | Ethanol |
| 1,5 | Polymerisat PF als wässrige Dispersion |
| 10,0 | Propan/Butan Wasser auf 100 g |

Die Komponenten werden gemischt und zusammen mit dem Treibmittel abgefüllt.

| Formulierung 45: | |
|---|---|
| 2,0 | Cocotrimoniummethosulfat |
| 0,2 | Parfümöl |
| 6,7 | Acrylsäure-Copolymer |
| 0,6 | Aminomethylpropanol |
| 2,5 | Polyvinylcaprolactam |
| 0,2 | Ceteareth 25 |
| 0,2 | Panthenol |
| 0,1 | PEG-25 PABA |
| 0,2 | Hydroxyethylcellulose |
| 15,0 | Ethanol |
| 1,0 | Polymerisat PF als wässrige Dispersion |
| 10,0 | Propan/Butan Wasser auf 100 g |

### Farbiges Haarshampoo

| Formulierung 46: | |
|---|---|
| 40,0 | Natriumlaurylsulfat |
| 10,0 | Cocoamidopropylbetain |
| q.s. | Parfümöl |
| 3,0 | Polyquaternium-44 |
| q.s. | Konservierungsmittel |
| 0,5 | Natriumchlorid |
| 1,5 | Polymerisat PF als wässrige Dispersion Wasser auf 100 g |

### Sonnenschutzcreme

| Formulierung 47: | |
|---|---|
| 1,5 | Ceteareth 6 |
| 1,0 | Cetanol |
| 3,0 | Cetearyloctanoat |
| 5,0 | Polymerpulver aus Beispiel 1 |
| 2,0 | Butylmethoxydibenzoylmethan |
| 6,0 | Isopropylstearat |
| 1,0 | Glycerylstearat |
| 2,0 | Stearinsäure |
| 3,0 | Polyethylenglykol 300 |
| 0,3 | Carbomer |
| 0,6 | Tetrahydroxypropylethylendiamin |
| 0,1 | Dinatrium-EDTA |
| 0,1 | Butylparaben |
| 0,2 | Methylparaben |
| 74,2 | Wasser |

## Patentansprüche

1. Farbmittelhaltige wässrige Polymerdispersion, enthaltend:
a. farbmittelhaltige Polymerisatteilchen PF mit einem mittleren Teilchendurchmesser d₅₀ unterhalb 1000 nm, umfassend:
i. eine aus ethylenisch ungesättigten Monomeren M aufgebaute Polymermatrix und
ii. wenigstens ein in der Polymermatrix homogen verteiltes organisches Farbmittel F, ausgewählt unter Farbstoffen, UV-Absorbern und optischen Aufhellern, in einer Menge von 0,5 bis 50 Gew.-%, bezogen auf die Polymermatrix;
b. wenigstens eine nichtionische oberflächenaktive Verbindung NO in einer Menge von 0,1 bis 20 Gew.-% bezogen auf die Polymermatrix; und
c. wenigstens ein amphiphiles Polymer PA, das 0,5 bis 10 mol/kg anionische funktionelle Gruppen aufweist, in einer Menge von 1 bis 50 Gew.-%, bezogen auf die Polymermatrix.

2. Polymerdispersion nach Anspruch 1, worin die nichtionische oberflächenaktive Verbindung wenigstens eine Alkylgruppe mit 6 bis 32 C-Atomen und wenigstens eine Oligoethergruppe der allgemeinen Formel -[CH₂CH₂O]ₙ-H aufweist, worin n wenigstens 2 ist.

3. Polymerdispersion nach Anspruch 1 oder 2, worin das amphiphile Polymer PA Carboxylat-Gruppen als anionische funktionelle Gruppen aufweist.

4. Polymerdispersion nach Anspruch 3, worin das Polymer PA einpolymerisiert enthält:
- 5 bis 70 Mol-% wenigstens eines Monomers A, ausgewählt unter monoethylenisch ungesättigten Mono- und Dicarbonsäuren mit 3 bis 8 C-Atomen;
- 30 bis 95 Mol-% wenigstens eines begrenzt wasserlöslichen oder wasserunlöslichen Monomers B und gegebenenfalls
- bis 30 Mol-% eines von den Monomeren A und B verschiedenen Monomers C, jeweils bezogen auf die Summe der Monomere A, B und C,
und worin bis 50 Mol-% der Carboxylgruppen durch eine oberflächenaktive Verbindung mit Oligoethergruppe der allgemeinen Formel -[CH₂CH₂O]ₙ-H, worin n wenigstens 2 ist, verestert sein können.

5. Polymerdispersion nach Anspruch 4, worin das Polymer PA ausgewählt ist unter den Salzen von:
- Copolymeren aus Maleinsäure als Monomer A und C₃-C₂₀-Olefinen als Monomer B, worin 1 bis 50 Mol-% der Carboxylgruppen durch eine oberflächenaktive Verbindung mit Oligoethergruppe der allgemeinen Formel -[CH₂CH₂O]ₙ-H, worin n wenigstens 2 ist, verestert sein können;
- Copolymeren monoethylenisch ungesättigter C₃-C₈-Monocarbonsäuren als Monomere A mit Vinylestern aliphatischer C₂-C₂₀-Carbonsäuren als Monomere B;
- Copolymeren aus Monomeren A mit Monomeren B, ausgewählt unter den C₁-C₂₀-Alkylestern monoethylenisch ungesättigter Monocarbonsäuren und den N-C₁-C₂₀-Alkyl- und N,N-(Di-C₁-C₂₀-alkyl)amiden monoethylenisch ungesättigter C₃-C₈-Monocarbonsäuren.

6. Polymerdispersion nach einem der vorhergehenden Ansprüche worin die Polymermatrix als Monomere M umfasst:
- 70 bis 99,9 Gew.-% wenigstens eines Monomers M1 mit einer Wasserlöslichkeit im Bereich von 0,01 g/l bis 80 g/l (bei 25°C und 1 bar),
- 0,1 bis 30 Gew.-% wenigstens eines vernetzenden Monomers M2, das wenigstens zwei nicht konjugierte ethylenisch ungesättigte Doppelbindungen aufweist, und
- 0,1 bis 20 Gew.-% eines oder mehrerer Monomere M3 mit einer Wasserlöslichkeit < 0,01 g/l (bei 25°C und 1 bar),
- 0 bis 30 Gew.-% davon verschiedene Monomere M4, wobei die Gewichtsanteile der Monomere M1 bis M4 jeweils auf 100 Gew.-% Monomere M bezogen sind.

7. Polymerdispersion nach einem der vorhergehenden Ansprüche, worin das Farbmittel F ein UV-Absorber ist.

8. Farbmittelhaltiges Polymerpulver, erhältlich durch Verdampfen der flüchtigen Bestandteile einer wässrigen farbmittelhaltigen Polymerdispersion gemäss einem der Ansprüche 1 bis 7.

9. Verfahren zur Herstellung einer farbmittelhaltigen Polymerdispersion gemäss einem der Ansprüche 1 bis 7, umfassend die aufeinanderfolgenden Schritte:
i. Lösen des Farbmittels F in den Monomeren M,
ii. Erzeugen einer konventionellen, farbmittelhaltigen Öl-in-Wasser-Emulsion durch Emulgieren der Monomer-Farbmittellösung in Wasser und
iii. Homogenisieren der konventionellen Emulsion zu einer farbmittelhaltigen Miniemulsion, worin die Monomertröpfchen einen mittleren Tröpfchendurchmesser unterhalb 1000 nm aufweisen,
iv. Polymerisation der Miniemulsion in Gegenwart eines die radikalische Polymerisation der Monomere M auslösenden Polymerisationsinitiators,
**dadurch gekennzeichnet, dass** die Polymerisation in Gegenwart von 0,1 bis 20 Gew.-% wenigstens einer nichtionischen oberflächenaktiven Verbindung NO und 1 bis 50 Gew.-%, jeweils bezogen auf die Monomere M, wenigstens eines amphiphilen Polymers PA durchgeführt wird.

10. Kosmetisches Mittel enthaltend wenigstens ein farbmittelhaltiges Polymerisat PF in Form einer wässrigen Polymerdispersion gemäss einem der Ansprüche 1 bis 7 und/oder ein Polymerpulver gemäss Anspruch 8 und für kosmetische Mittel übliche Adjuvantien.

11. Kosmetisches Mittel in Form einer Sonnenschutzformulierung, enthaltend wenigstens ein UV-Absorber enthaltendes Polymerisat PF in Form einer wässrigen Polymerdispersion gemäss Anspruch 7 und/oder ein daraus hergestelltes Polymerpulver.

## Claims

1. A colorant-containing aqueous polymer dispersion comprising:
a. colorant-containing polymer particles PC having an average particle diameter d₅₀ below 1000 nm, comprising:
i. a polymer matrix constructed from ethylenically unsaturated monomers M and
ii. at least one organic colorant C homogeneously distributed within the polymer matrix and chosen from dyes, UV absorbers and optical brighteners, in an amount of from 0.5 to 50% by weight, based on the polymer matrix;
b. at least one nonionic surface-active compound NS in an amount of from 0.1 to 20% by weight, based on the polymer matrix; and
c. at least one amphiphilic polymer PA which has 0.5 to 10 mol/kg of anionic functional groups, in an amount of from 1 to 50% by weight, based on the polymer matrix.

2. A polymer dispersion as claimed in claim 1, in which the nonionic surface-active compound has at least one alkyl group having 6 to 32 carbon atoms and at least one oligoether group of the formula -[CH₂CH₂O]ₙ-H, in which n is at least 2.

3. A polymer dispersion as claimed in claim 1 or 2, in which the amphiphilic polymer PA has carboxylate groups as anionic functional groups.

4. A polymer dispersion as claimed in claim 3, in which the polymer PA comprises, in copolymerized form:
- 5 to 70 mol% of at least one monomer A, chosen from monoethylenically unsaturated mono- and dicarboxylic acids having 3 to 8 carbon atoms;
- 30 to 95 mol% of at least one monomer B which is insoluble or has limited solubility in water, and optionally
- up to 30 mol% of a monomer C which is different from monomers A and B, in each case based on the sum of the monomers A, B and C,
and in which up to 50 mol% of the carboxyl groups can be esterified by a surface-active compound with oligoether group of the formula -[CH₂CH₂O]ₙ-H, in which n is at least 2.

5. A polymer dispersion as claimed in claim 4, in which the polymer PA is chosen from the salts of:
- copolymers of maleic acid as monomer A and C₃-C₂₀-olefins as monomer B, in which 1 to 50 mol% of the carboxyl groups can be esterified by a surface-active compound containing oligoether groups of the formula -[CH₂CH₂O]ₙ-H, in which n is at least 2;
- copolymers of monoethylenically unsaturated C₃-C₈-monocarboxylic acids as monomer A with vinyl esters of aliphatic C₂-C₂₀-carboxylic acids as monomer B;
- copolymers of monomers A and monomers B, chosen from the C₁-C₂₀-alkyl esters of monoethylenically unsaturated monocarboxylic acids and the N-C₁-C₂₀-alkyl- and N,N-(di-C₁-C₂₀-alkyl)amides of monoethylenically unsaturated C₃-C₈-monocarboxylic acids.

6. A polymer dispersion as claimed in any of the preceding claims, in which the polymer matrix comprises, as monomers M:
- 70 to 99.9% by weight of at least one monomer M1 having a solubility in water in the range from 0.01 g/l to 80 g/l (at 25°C and 1 bar),
- 0.1 to 30% by weight of at least one crosslinking monomer M2 which has at least two nonconjugated ethylenically unsaturated double bonds, and
- 0.1 to 20% by weight of one or more monomers M3 having a solubility in water of < 0.01 g/l (at 25°C and 1 bar),
- 0 to 30% by weight of monomers M4 different therefrom, where the weight proportions of the monomers M1 to M4 are in each case based on 100% by weight of monomers M.

7. A polymer dispersion as claimed in any of the preceding claims, in which the colorant C is a UV absorber.

8. A colorant-containing polymer powder obtainable by evaporating the volatile constituents of an aqueous colorant-containing polymer dispersion as claimed in any of claims 1 to 7.

9. A process for the preparation of a colorant-containing polymer dispersion as claimed in any of claims 1 to 7, comprising the successive steps:
i. dissolution of the colorant C in the monomers M,
ii. production of a conventional, colorant-containing oil-in-water emulsion by emulsifying the monomer/colorant solution in water and
iii.homogenization of the conventional emulsion to give a colorant-containing miniemulsion in which the monomer droplets have an average droplet diameter below 1000 nm,
iv. polymerization of the miniemulsion in the presence of a polymerization initiator which triggers the free-radical polymerization of the monomers M,
which comprises carrying out the polymerization in the presence of from 0.1 to 20% by weight of at least one nonionic surface-active compound NS and 1 to 50% by weight, in each case based on the monomers M, of at least one amphiphilic polymer PA.

10. A cosmetic composition comprising at least one colorant-containing polymer PC in the form of an aqueous polymer dispersion as claimed in any of claims 1 to 7 and/or a polymer powder as claimed in claim 8 and adjuvants customary for cosmetic compositions.

11. A cosmetic composition in the form of a sunscreen formulation, comprising at least one UV-absorber-containing polymer PC in the form of an aqueous polymer dispersion as claimed in claim 7 and/or a polymer powder prepared therefrom.

## Revendications

1. Dispersion aqueuse de polymères contenant des colorants, et constituée de:
a. des particules de polymère contenant des colorants, à un diamètre de particule moyen d₅₀ inférieur à 1000 nm, et constituées de :
i. une gangue polymère formée de monomères à insaturation éthylénique M et
ii. au moins un colorant organique F en répartition homogène dans la gangue polymère, ce colorant étant en fait choisi parmi les colorants, les absorbeurs d'UV et les azureurs optiques, en quantité de 0,5 à 5% du poids de la gangue polymère ;
b. au moins un composé tensio-actif non-ionique NO en quantité de 0,1 à 20% du poids de la gangue polymère ; et
c. au moins un polymère amphiphile PA contenant 0,5 à 10 mol/kg de groupes fonctionnels anioniques, en quantité de 1 à 50% du poids de la gangue polymère.

2. Dispersion de polymères selon la revendication 1, dans laquelle le composé tensio-actif non-ionique contient au moins un groupe alkyle en C₆-C₃₂ et au moins un groupe oligoéther de formule générale -[CH₂CH₂O]ₙ-H dans laquelle n est égal au moins à 2.

3. Dispersion de polymères selon la revendication 1 ou 2, dans laquelle le polymère amphiphile PA contient, en tant que groupes fonctionnels anioniques, des groupes carboxylate.

4. Dispersion de polymères selon la revendication 3, dans laquelle le polymère PA contient, à l'état polymérisé :
- 5 à 70 mol % d'au moins un monomère A choisi parmi les acides mono- et di-carboxyliques à insaturation monoéthylénique en C₃-C₈ ;
- 30 à 95 mol % d'au moins un monomère B insoluble dans l'eau ou à solubilité limitée dans l'eau et le cas échéant,
- jusqu'à 30 mol % d'un monomère C autre que les monomères A et B, ces proportions molaires se rapportant à la somme des monomères A, B et C, et dans lequel jusqu'à 50 mol % des groupes carboxyle peuvent être estérifiés par un composé tensio-actif à groupes oligoéther de formule générale - [CH₂CH₂O]ₙ-H dans laquelle n est égal au moins à 2.

5. Dispersion de polymères selon la revendication 4, dans laquelle le polymère PA est choisi parmi les sels de :
- les copolymères de l'acide maléique, constituant le monomère A, et d'oléfines en C₃-C₂₀ constituant les monomères B, 1 à 50 mol % des groupes carboxyle pouvant être estérifiés par un composé tensio-actif à groupes oligoéther de formule générale -[CH₂CH₂O]ₙ-H dans laquelle n est égal au moins à 2 ;
- les copolymères d'acides monocarboxyliques à insaturation monoéthylénique en C₃-C₈, constituant les monomères A, et d'esters vinyliques d'acides carboxyliques aliphatiques en C₂-C₂₀ constituant les monomères B ;
- les copolymères de monomères A et de monomères B choisis parmi les esters alkyliques en C₁-C₂₀ d'acides monocarboxyliques à insaturation monoéthylénique et les N-(alkyle en C₁-C₂₀)- et N,N-di-(alkyle en C₁-C₂₀)amides d'acides monocarboxyliques à insaturation monoéthylénique en C₃-C₈.

6. Dispersion de polymères selon l'une des revendications qui précèdent dans laquelle la gangue polymère contient en tant que monomères M :
- 70 à 99,9% en poids d'au moins un monomère M1 ayant une solubilité dans l'eau qui va de 0,01 g/l à 80 g/l (à 25°C sous 1 bar),
- 0,1 à 30% en poids d'au moins un monomère réticulant M2 qui contient au moins deux doubles liaisons éthyléniques non conjuguées et
- 0,1 à 20% en poids d'un ou plusieurs monomères M3 ayant une solubilité dans l'eau inférieure à 0,01 g/l (à 25°C sous 1 bar),
- 0 à 30% en poids d'autres monomères M4, les proportions en poids indiquées pour les monomères M1 à M4 se rapportant à 100% en poids des monomères M.

7. Dispersion de polymères selon l'une des revendications qui précèdent dans laquelle le colorant F est un absorbeur UV.

8. Poudre de polymères contenant des colorants, obtenue par vaporisation des constituants volatils d'une dispersion aqueuse de polymères contenant des colorants selon l'une des revendications 1 à 7.

9. Procédé pour la préparation d'une dispersion de polymères contenant des colorants selon l'une des revendications 1 à 7, comprenant les stades opératoires successifs suivants :
i. dissolution du colorant F dans les monomères M,
ii. formation d'une émulsion de type classique, huile-dans-l'eau, contenant des colorants, par émulsion dans l'eau de la solution de colorant dans les monomères,
iii. homogénéisation de l'émulsion obtenue en une mini-émulsion contenant des colorants, les gouttelettes des monomères ayant un diamètre moyen inférieur à 1000 nm,
iv. polymérisation de la mini-émulsion en présence d'un inducteur de polymérisation déclenchant la polymérisation radicalaire des monomères M,
**caractérisé en ce que** la polymérisation est réalisée en présence de 0,1 à 20% en poids d'au moins un composé tensio-actif non-ionique NO et de 1 à 50% en poids, ces pourcentages se rapportant aux monomères M, d'au moins un polymère amphiphile PA.

10. Produit cosmétique contenant au moins un polymère contenant des colorants PF à l'état de dispersion aqueuse selon l'une des revendications 1 à 7 et/ou une poudre de polymère selon la revendication 8 et les adjuvants usuels pour les produits cosmétiques.

11. Produit cosmétique sous forme d'une composition antisolaire contenant un polymère PF qui contient lui-même un absorbeur d'UV sous forme d'une dispersion aqueuse de polymère selon la revendication 7 et/ou d'une poudre de polymère préparée à partir de cette dernière.
